(19) Europäisches Patentamt
European Patent Office
Office européen des brevets

(11) Veröffentlichungsnummer: **0 387 618 B1**

(12) **EUROPÄISCHE PATENTSCHRIFT**

(45) Veröffentlichungstag der Patentschrift: **27.07.94**

(21) Anmeldenummer: **90103990.9**

(22) Anmeldetag: **01.03.90**

Die Akte enthält technische Angaben, die nach dem Eingang der Anmeldung eingereicht wurden und die nicht in dieser Patentschrift enthalten sind.

(51) Int. Cl.⁵: **C07D 471/06**, C07D 498/06, C07D 513/06, A61K 31/55, //(C07D471/00,221:00,209:00), (C07D498/00,265:00,209:00), (C07D513/00,279:00,209:00)

# (54) 1,7-anellierte 1H-Indol-2-carbonsäure-N-(1,4-benzodiazepin-3-yl)amide.

(30) Priorität: **08.03.89 DE 3907389**
**08.03.89 DE 3907390**

(43) Veröffentlichungstag der Anmeldung:
**19.09.90 Patentblatt 90/38**

(45) Bekanntmachung des Hinweises auf die Patenterteilung:
**27.07.94 Patentblatt 94/30**

(84) Benannte Vertragsstaaten:
**AT BE CH DE DK ES FR GB GR IT LI LU NL SE**

(56) Entgegenhaltungen:
**EP-A- 0 139 584**
**EP-A- 0 284 256**
**EP-A- 0 322 016**
**FR-A- 2 567 126**
**US-A- 4 628 084**

(73) Patentinhaber: **Kali-Chemie Pharma GmbH**
**Hans-Böckler-Allee 20**
**D-30173 Hannover(DE)**

(72) Erfinder: **Waldeck, Harald**
**Alte Döhrener Strasse 6**
**D-3000 Hannover 1(DE)**
Erfinder: **Benson, Werner**
**OT Letter**
**D-3016 Seelze 2(DE)**
Erfinder: **Zeugner, Horst**
**Havelweg 10**
**D-3000 Hannover 73(DE)**
Erfinder: **Wolf, Klaus-Ullrich**
**Imkersweg 6**
**D-3162 Uetze(DE)**
Erfinder: **Gregory, Peter-Colin**
**Molanusweg 34**
**D-3000 Hannover 71(DE)**
Erfinder: **Hamminga, Derk**
**Walstro 37**
**NL-3831 WV Leusden(NL)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 387 618 B1

**CHEMICAL ABSTRACTS, Band 105, Nr. 13, 29. September 1986, Seite 17, Zusammenfassung-Nr. 107944z, Columbus, Ohio, US; B.E. EVANS et al.: "Design of potent, orally effective, nonpeptidal antagonists of the peptide hormone cholecystokinin"**

**CHEMICAL ABSTRACTS, Band 109, Nr. 23, 5. Dezember 1988, Seite 16, Zusammenfassung-Nr. 204385h, Columbus, Ohio, US; B.E. EVANS et al.: "Methods of drug discovery: development of potent, selective, orally effective cholecystokinin antagonists"**

**JOURNAL OF HETEROCYCLIC CHEMISTRY, Band 11, Nr. 3, 1974, Seiten 387-393; E.A. STECK et al.: "Some 5,6-dihydro-4H-pyrrolo-(3,2,1-i,j)quinolines"**

Erfinder: **van Wijngaarden, Ineke**
**Dennenlaan 18**
**B-2360 Oud-Turnhout(BE)**

(74) Vertreter: **Lauer, Dieter, Dr.**
**c/o Kali-Chemie Aktiengesellschaft**
**Hans-Böckler-Allee 20**
**D-30173 Hannover (DE)**

## Beschreibung

Die vorliegende Erfindung betrifft neue Amide von 3-Amino-1,4-benzodiazepinderivaten mit 1,7-anellierten 1H-Indol-2-carbonsäurederivaten und deren Salze sowie diese Verbindungen enthaltende pharmazeutische Zubereitungen und Verfahren zur Herstellung dieser Verbindungen.

Aus EP-A-0 284 256, EP-A-0 167 919, US-A-4 628 084, Chem. Abstracts 105, 107 944z und Chem. Abstracts 109, 204 385h sind in 3-Stellung durch Acylaminoreste substituierte 2-Oxo-5-phenyl-1,4-benzodiazepinderivate mit CCK-antagonistischen Wirkungen bekannt.

Cholecystokinin (=CCK) ist ein im gastrointestinalen Gewebe und im zentralen Nervensystem vorkommendes Peptid mit einem weit gefächerten Wirkungsspektrum, welches unter anderem stimulierende Wirkungen auf die Colonmotilität, die Gallenblasenkontraktion und die exokrine Pankreassekretion und hemmende Wirkungen auf die Magenentleerung ausübt und auch Einfluß auf die Appetitregulierung besitzt. CCK-Antagonisten sind pharmakologisch wirksame Substanzen, welche ein Bindungsvermögen an CCK-Rezeptoren besitzen und somit CCK-induzierte Vorgänge hemmen können.

Aufgabe der vorliegenden Erfindung ist es, neue CCK-antagonistisch wirksame Verbindungen mit einem verbesserten Wirkungsprofil zu entwickeln.

Ferner liegt der Erfindung die Aufgabe zugrunde, neue Derivate von 1,7-anellierten 1H-Indol-2-carbonsäuren mit wertvollen pharmakologischen Eigenschaften herzustellen.

Es wurde nun gefunden, daß die erfindungsgemäßen 1,7-anellierten 1H-Indol-2-carbonsäure-N-(1,4-benzodiazepin-3-yl)-amide CCK-antagonistische Eigenschaften besitzen und sich durch ein neuartiges pharmakologisches Wirkungsprofil mit ausgeprägter die Magenentleerung fördernder Wirkungskomponente bei guter therapeutischer Breite und geringer Toxizität auszeichnen.

Die vorliegende Erfindung betrifft daher neue 1,7-anellierte 1H-Indol-2-carbonsäure-N-(1,4-benzodiazepin-3-yl)-amid-Verbindungen der allgemeinen Formel I

(s. Formel I)

worin

$R^1$ Wasserstoff, Alkyl mit 1-4 Kohlenstoffatomen oder Cycloalkylalkyl mit 4-7 Kohlenstoffatomen bedeutet,

$R^2$ Wasserstoff, Halogen, Alkyl mit 1-5 Kohlenstoffatomen, Alkoxy mit 1-5 Kohlenstoffatomen oder Trifluormethyl bedeutet und

$R^3$ Wasserstoff, Halogen, Alkyl mit 1-5 Kohlenstoffatomen oder Alkoxy mit 1-5 Kohlenstoffatomen bedeutet oder

$R^2$ und $R^3$ an zwei benachbarte Kohlenstoffatome gebunden sind und gemeinsam eine Alkylendioxygruppe mit 1-2 Kohlenstoffatomen bedeuten,

$R^4$ für Cycloalkyl mit 5 bis 6 Kohlenstoffatomen, Thiophen oder eine gegebenenfalls substituierte Phenylgruppe a steht,

(s. Formel a)

worin

$R^7$ Wasserstoff, Halogen, Alkyl mit 1-5 Kohlenstoffatomen, Alkoxy mit 1-5 Kohlenstoffatomen oder Trifluormethyl und

$R^8$ Wasserstoff, Halogen, Alkyl mit 1-5 Kohlenstoffatomen oder Alkoxy mit 1-5 Kohlenstoffatomen bedeuten,

$R^5$ Wasserstoff oder Halogen bedeutet,

$R^6$ Wasserstoff, Halogen, Alkyl mit 1-5 Kohlenstoffatomen, Alkoxy mit 1-5 Kohlenstoffatomen oder Trifluormethyl bedeutet und

Z für eine Alkylenkette mit 2-4 Kohlenstoffatomen, welche gegebenenfalls durch Alkyl mit 1-4 Kohlenstoffatomen mono- oder disubstituiert sein kann oder an welche gegebenenfalls ein 5-6-gliedriger carbocyclischer Ring anelliert sein kann, oder für eine $-X-CH_2-CH_2-$kette, worin X an den Phenylring des Indolgerüstes gebunden ist und Sauerstoff oder Schwefel bedeutet, steht,

sowie deren Säureadditionssalze.

In den Verbindungen der Formel I steht $R^1$ vorzugsweise für eine Alkylgruppe. Diese kann geradkettig oder verzweigt sein und 1-4 Kohlenstoffatome enthalten. Als Beispiel einer Cycloalkylalkylgruppe sei Cyclopropylmethyl genannt. Als besonders geeignete Reste $R^1$ haben sich geradkettige und verzweigte Alkylgruppen mit 1-3 Kohlenstoffatomen, insbesondere Methyl erwiesen.

Sofern in den Verbindungen der Formel I die Substituenten $R^2$, $R^3$ und $R^6$ eine Alkylgruppe darstellen oder enthalten, kann diese eine gerade oder verzweigte Alkylgruppe mit 1-5, vorzugsweise 1-4 Kohlenstoffatomen, insbesondere Methyl oder Äthyl darstellen. So stellen Alkylsubstituenten bevorzugt Methyl und

Alkoxysubstituenten bevorzugt Methoxy dar. Halogensubstituenten $R^1$, $R^2$, $R^3$, $R^5$ und $R^6$ stellen insbesondere Fluor, Chlor oder Brom dar.

Die Substituenten $R^2$ und $R^3$ sind vorzugsweise in 7- und 8-Stellung des Benzodiazepingerüstes angeordnet und stellen vorzugsweise Wasserstoff, Alkoxy, insbesondere Methoxy, oder Alkyl, insbesondere Methyl, oder auch Chlor dar. So erweist sich insbesondere ein Methoxy-Substituent in 8-Stellung als günstig.

Der Substituent $R^4$ stellt vorzugsweise eine gegebenenfalls substituierte Phenylgruppe dar. Die Substituenten $R^7$ und $R^8$ der 5-Phenylgruppe stellen vorzugsweise Wasserstoff, Alkyl, insbesondere Methyl, oder Halogen, insbesondere Fluor oder Chlor, oder auch Alkoxy mit 1-5 Kohlenstoffatomen, beispielsweise Isopentyloxy, dar. So eignet sich insbesondere eine unsubstituierte oder eine durch Fluor substituierte Phenylgruppe $R^4$.

Falls der Substituent $R^4$ eine Cycloalkylgruppe darstellt, ist diese bevorzugt Cyclohexyl.

Der Substituent $R^6$ stellt vorzugsweise Wasserstoff oder auch Halogen, insbesondere Fluor, oder Alkoxy, insbesondere Methoxy, dar. Der Substituent $R^5$ stellt vorzugsweise Wasserstoff dar. Falls $R^5$ Halogen bedeutet, ist dieses vorzugsweise Chlor.

Z stellt eine Kette mit 2-4 Kettengliedern, vorzugsweise eine Alkylenkette mit 2-4 Kohlenstoffatomen dar. Z bildet somit mit der Aminoäthylengruppierung, an welches es gebunden ist, einen 5- bis 7-gliedrigen Heterocyclus. Vorzugsweise stellt Z eine Propylenkette dar und bildet so gemeinsam mit dem Indolgerüst, an welches es gebunden ist, ein 4H-Pyrrolo[3,2,1-ij]-5,6-dihydrochinolin-Gerüst. Sofern die Alkylenkette Z durch Alkyl substituiert ist, kann dieses 1-4 Kohlenstoffatome enthalten und stellt insbesondere Methyl dar. Sofern an die Alkylenkette Z ein carbocyclischer Ring anelliert ist, kann dieser ungesättigt oder gesättigt sein und stellt vorzugsweise einen Benzolring dar.

Die Verbindungen der Formel I enthalten ein chirales Kohlenstoffatom in 3-Stellung des Benzodiazepingerüstes und können in der D- und der L-Form oder als Racemat vorliegen. Die vorliegende Erfindung umfaßt sowohl die racemischen Gemische wie auch die reinen optischen Isomeren der Verbindungen der Formel I.

Erfindungsgemäß werden die neuen Amide der Formel I und deren Säureadditionssalze erhalten, indem man in an sich bekannter Weise Amino-Verbindungen der allgemeinen Formel II

(s. Formel II)

worin $R^1$, $R^2$, $R^3$ und $R^4$ obige Bedeutung besitzen, mit Säuren oder reaktionsfähigen Säurederivaten der allgemeinen Formel III

(s. Formel III)

worin $R^5$, $R^6$ und Z obige Bedeutung besitzen und Y Hydroxy oder eine reaktive Gruppe bedeutet, acyliert und gegebenenfalls freie Verbindungen der Formel I in ihre Säureadditionssalze überführt oder die Säureadditionssalze in die freien Verbindungen der Formel I überführt.

Die Acylierung der Amino-Verbindungen der Formel II kann nach an sich zur Bildung von Amidgruppierungen durch Aminoacylierung üblichen Methoden ausgeführt werden. Als Acylierungsmittel können die Säuren der Formel IIIa

(s. Formel IIIa)

worin $R^6$, $R^7$ und Z obige Bedeutung besitzen oder deren reaktionsfähige Derivate eingesetzt werden. Als reaktionsfähige Derivate kommen insbesondere gemischte Säureanhydride, Ester und Säurehalogenide in Frage. So können reaktive Gruppen Y beispielsweise niederes Alkoxy, Halogene wie Chlor oder Brom oder vorzugsweise organische Sulfonsäurereste darstellen, beispielsweise Reste von Niederalkansulfonsäuren wie z.B. Methansulfonsäure oder von aromatischen Sulfonsäuren wie Benzolsulfonsäure oder durch niederes Alkyl oder Halogen substituierten Benzolsulfonsäuren, z.B. Toluolsulfonsäuren oder Brombenzolsulfonsäuren. Die Acylierung kann in einem unter den Reaktionsbedingungen inerten organischen Lösungsmittel, vorzugsweise bei Temperaturen zwischen -20 °C und Raumtemperatur erfolgen. Als Lösungsmittel eignen sich insbesondere halogenierte Kohlenwasserstoffe wie Dichlormethan oder aromatische Kohlenwasserstoffe wie Benzol oder Toluol oder cyclische Äther wie Tetrahydrofuran oder Dioxan oder Gemische dieser Lösungsmittel.

Die Acylierung kann zweckmäßig, insbesondere wenn als Acylierungsmittel ein gemischtes Anhydrid der Säuren der Formel IIIa mit einer Sulfonsäure verwendet wird, in Gegenwart eines säurebindenden Reagenzes durchgeführt werden. Als säurebindende Mittel eignen sich in dem Reaktionsgemisch lösliche Basen, insbesondere organische Basen wie tert. Niederalkylamine und Pyridine wie z.B. Triäthylamin, Tripropylamin, Pyridin, 4-Dimethylaminopyridin, 4-Diäthylaminopyridin oder 4-Pyrrolidinopyridin. Im Überschuß eingesetzte organische Basen können gleichzeitig auch als Lösungsmittel dienen.

Vorteilhaft können gemischte Säureanhydride der Säuren der Formel IIIa mit organischen Sulfonsäuren in situ durch Umsetzen der Säuren IIIa mit einem Säurehalogenid, insbesondere dem Säurechlorid der

organischen Sulfonsäure erhalten werden und ohne Isolierung direkt weiter mit der Aminoverbindung der Formel II umgesetzt werden.

Falls als Acylierungsmittel die Säure selbst oder auch ein Ester eingesetzt wird, kann die Umsetzung der Aminoverbindung der Formel II mit der Säure der Formel IIIa oder deren Ester zweckmäßig auch in Gegenwart eines aus der Peptidchemie als zur Amidbildung geeignet bekannten Kopplungsreagenzes durchgeführt werden. Als Beispiel von Kopplungsreagenzien, welche die Amidbildung mit den freien Säuren dadurch fördern, daß sie mit der Säure in situ unter Bildung eines reaktionsfähigen Säurederivates reagieren, seien insbesondere genannt Alkylcarbodiimide, z.B. Cycloalkylcarbodiimide wie Dicyclohexylcarbodiimid, oder 1-Äthyl-3-[3-(dimethylamino)-propyl]-carbodiimid, Carbonyldiimidazol und N-Niederalkyl-2-halogenpyridiniumsalze, insbesondere Halogenide oder Tosylate, vorzugsweise N-Methyl-2-chlorpyridiniumjodid (s. z.B. Mukaiyama in Angew. Chemie 91 789-812). Die Umsetzung in Gegenwart eines Kopplungsreagenzes kann zweckmäßig bei Temperaturen von -30 °C bis +50 °C unter Benutzung von Lösungsmitteln wie halogenierten Kohlenwasserstoffen und/oder aromatischen Lösungsmitteln gegebenenfalls in Gegenwart eines säurebindenden Amins durchgeführt werden.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden. Als pharmakologisch annehmbare Säureadditionsssalze der Verbindungen der Formel I eignen sich beispielsweise deren Salze mit anorganischen Säuren z.B. Halogenwasserstoffsäuren, insbesondere Chlorwasserstoffsäure, Schwefelsäure oder Phosphorsäuren oder mit organischen Säuren, beispielsweise niederen aliphatischen Mono- oder Dicarbonsäuren wie Milchsäure, Maleinsäure, Fumarsäure, Weinsäure oder Essigsäure oder Sulfonsäuren, beispielsweise Niederalkylsulfonsäuren wie Methansulfonsäure oder gegebenenfalls im Benzolring durch Halogen oder niederes Alkyl substituierte Benzolsulfonsäuren wie p-Toluolsulfonsäure oder Cyclohexylaminosulfonsäure.

Falls bei der Synthese Racemate der Verbindungen der Formel II eingesetzt werden, werden die Verbindungen der Formel I in Form von Racematen erhalten. Ausgehend von optisch aktiven Formen der Verbindungen der Formel II können optisch aktive Verbindungen der Formel I erhalten werden. Die optisch aktiven Verbindungen der Formel I können aus den racemischen Gemischen in an sich bekannter Weise erhalten werden, z.B. durch chromatographische Trennung an chiralen Trennmaterialien oder durch Umsetzung mit geeigneten optisch aktiven Säuren, beispielsweise Weinsäure oder 10-Camphersulfonsäure und anschließende Auftrennung in ihre optisch aktiven Antipoden durch fraktionierte Kristallisation der gewonnenen Salze.

Die Aminverbindungen der Formel II sind bekannt oder können nach an sich bekannten Verfahren oder analog zu an sich bekannten Verfahren hergestellt werden. So können die Aminverbindungen der Formel II z.B. auf an sich bekannte Weise durch Reduktion einer Oxim-Verbindung der Formel IV

(s. Formel IV)

worin $R^1$, $R^2$, $R^3$ und $R^4$ obige Bedeutung besitzen, erhalten werden. Die Reduktion der Oxime der Formel IV zu den Aminen der Formel II kann nach üblichen Methoden, beispielsweise katalytischer Hydrierung vorzugsweise in Gegenwart eines Raney-Nickel-Katalysators oder mit Zink/Eisessig als Reduktionsmittel erfolgen. Bei der Reduktion mit Zink/Eisessig kann es sich als vorteilhaft erweisen, zur Aktivierung des Zinks eine halogenierte organische Carbonsäure zuzusetzen.

Die Verbindungen der Formel II enthalten in 3-Stellung des Benzodiazepingerüstes ein chirales Kohlenstoffatom. Sie werden bei der Synthese in Form von Racematen erhalten. Die optisch aktiven Verbindungen können aus den racemischen Gemischen in an sich bekannter Weise erhalten werden, z.B. durch chromatographische Trennung an chiralen Trennmaterialien oder durch Umsetzung mit geeigneten optisch aktiven Säuren, beispielsweise Weinsäure oder 10-Camphersulfonsäure, und anschließende Auftrennung in ihre optisch aktiven Antipoden durch fraktionierte Kristallisation der gewonnenen Salze. Racemische Gemische der Amine der Formel II können zur Auftrennung auch zunächst mit einer optisch aktiven Aminosäure, z.B. Phenylalanin, nach in der Peptidchemie üblichen Methoden zu den entsprechenden Amiden der optisch aktiven Aminosäure umgesetzt werden. So können die racemischen Verbindungen der Formel II beispielsweise mit einer Aminosäure, deren $NH_2$-Gruppe durch eine aus der Peptidchemie bekannte Schutzgruppe, z.B. tert. Butoxycarbonyl (= BOC-Gruppe), geschützt ist, umgesetzt und die Schutzgruppe anschließend in an sich bekannter Weise wieder abgespalten werden. Das dabei entstehende diastereomere Amidpaar kann auf an sich bekannte Weise, z.B. durch fraktionierte Kristallisation oder Chromatographie aufgetrennt werden und anschließend die Aminoverbindung der Formel II aus den Amiden in an sich bekannter Weise wieder freigesetzt werden.

Die Oximverbindungen der Formel IV können auf an sich bekannte Weise durch Nitrosierung von Verbindungen der Formel V

(s. Formel V)

worin $R^1$, $R^2$, $R^3$, und $R^4$ obige Bedeutung besitzen, hergestellt werden. Zweckmäßig werden die in 3-Stellung unsubstituierten Verbindungen der Formel V zunächst in einem unter den Reaktionsbedingungen inerten organischen Lösungsmittel beispielsweise einem aromatischen Kohlenwasserstoff wie Benzol oder Toluol oder einem cyclischen Äther wie Tetrahydrofuran mit einer starken Base, beispielsweise einem Alkalimetallalkoholat wie Kalium-tert. Butylat behandelt und sodann mit einem Nitrosierungsmittel, beispielsweise einem Niederalkylnitrit wie Isoamylnitrit oder tert. Butylnitrit umgesetzt.

Verbindungen der allgemeinen Formel Va

(s. Formel Va)

worin $R^{1'}$ die für $R^1$ angegebene Bedeutung mit Ausnahme von Wasserstoff besitzt und $R^2$, $R^3$ und $R^4$ obige Bedeutung besitzen, können auf an sich bekannte Weise erhalten werden, indem man 2-Chlormethyl-1,4-benzodiazepin-Verbindungen der Formel VI

(s. Formel VI)

worin $R^{1'}$, $R^2$, $R^3$ und $R^4$ obige Bedeutung besitzen, auf an sich bekannte Weise oxidiert. Die Oxidation kann beispielsweise durch Behandeln der Verbindungen der Formel VI mit einem geeigneten Oxidationsmittel in Gegenwart eines unter den Reaktionsbedingungen inerten Lösungsmittels erfolgen. Als Oxidationsmittel können beispielsweise Kaliumpermanganat, Chromtrioxid oder Dichromatsalze eingesetzt werden. Als gegenüber diesen Oxidationsmitteln inerte Lösungsmittel eignen sich beispielsweise halogenierte Kohlenwasserstoffe wie Dichlormethan, Wasser oder Essigsäure oder deren Gemische.

Die Ausgangsverbindungen der Formel VI sind bekannt oder können nach an sich bekannten Methoden oder analog zu an sich bekannten Methoden hergestellt werden.

Die Verbindungen können z.B. in bekannter Weise nach den in den deutschen Offenlegungsschriften Nr. 22 21 558 oder 25 20 937 beschriebenen Methoden ausgehend von 2-Hydroxy-1,3-diaminopropan-Verbindungen der allgemeinen Formel VIII

(s. Formel VIII)

worin $R^{1'}$, $R^2$, $R^3$ und $R^4$ obige Bedeutung besitzen, erhalten werden. Die Verbindungen der Formel VIII werden durch Behandeln mit Phosphoroxidtrichlorid cyclisiert. Zweckmäßigerweise behandelt man dazu die Verbindungen der Formel VIII oder deren Säureadditionssalze mit Phorphoroxidtrichlorid bei einer Temperatur zwischen 100 und 150 °C, vorzugsweise bei Siedetemperatur des Reaktionsgemisches. Hierbei wird ein Gemisch einer 2-Chlormethyl-1,4-benzodiazepin-Verbindung der Formel VI mit der dazu isomeren 3-Chlor-1,5-benzodiazocin-Verbindung der Formel VII

(s. Formel VII)

worin $R^{1'}$, $R^2$, $R^3$ und $R^4$ obige Bedeutung besitzen, erhalten. Die Benzodiazocin-Verbindung der Formel VII in dem Gemisch kann auf an sich bekannte Weise, zum Beispiel durch Erhitzen des Gemisches in einem unter den Reaktionsbedingungen inerten organischen Lösungsmittel, beispielsweise einem höher siedenden Halogenkohlenwasserstoff wie Tetrachloräthan, in die dazu isomere Verbindung der Formel VI umgelagert werden.

Zur Herstellung von 2-Hydroxy-1,3-diaminopropan-Verbindungen der Formel VIII kann von Anilinen der Formel IX

(s. Formel IX)

worin $R^2$ und $R^3$ obige Bedeutung besitzen, ausgegangen werden. In diesen Anilinen wird zunächst auf an sich bekannte Weise die Aminogruppe durch eine $R^{1'}$-Gruppe monosubstituiert und anschließend analog der in DE-OS 28 10 349 beschriebenen Methode mit 1,2-Epoxypropylphthalimid oder zunächst mit Epichlorhydrin und nachfolgend mit Phthalimid umgesetzt. Anschließend wird die Phthalimidgruppe in an sich bekannter Weise gespalten und die erhaltenen Verbindungen der Formel X

(s. Formel X)

worin $R^{1'}$, $R^2$ und $R^3$ obige Bedeutung besitzen, werden mit Säurehalogeniden der Formel XI

(s. Formel XI)

worin $R^4$ obige Bedeutung besitzt, acyliert.

Verbindungen der Formel V können auch ausgehend von Ketonen der allgemeinen Formel XII

(s. Formel XII)

worin $R^1$, $R^2$, $R^3$ und $R^4$ obige Bedeutung besitzen, auf an sich bekannte Weise erhalten werden, indem man diese mit einem Halogenessigsäurehalogenid zu Verbindungen der Formel XIII (s. Formel XIII)

(s. Formel XIII)

worin $R^1$, $R^2$, $R^3$ und $R^4$ obige Bedeutung besitzen und Hal Chlor oder Brom bedeutet, umsetzt und diese anschließend mit Ammoniak zu Verbindungen der Formel V kondensiert.

Die Ketone der Formel XII sind bekannt oder können nach an sich bekannten Verfahren oder analog zu an sich bekannten Verfahren hergestellt werden, zum Beispiel durch Umsetzung von p-substituierten

Anilinen der Formel IX mit Säurehalogeniden der Formel XI, beispielsweise in einer Friedel-Craft-Reaktion mit anschließender Hydrolyse, oder ausgehend von Anthranilsäuren, indem diese zunächst mit Essigsäureanhydrid kondensiert werden, das Kondensationsprodukt in einer Grignard-Reaktion mit Verbindungen der allgemeinen Formel XIV

(s. Formel XIV)

worin $R^4$ obige Bedeutung besitzt, zu den N-Acetylderivaten der Verbindungen der Formel XII umgesetzt und diese hydrolisiert werden.

Verbindungen der Formel V, worin $R^1$ Wasserstoff bedeutet, können auf an sich bekannte Weise zu anderen Verbindungen der Formel V alkyliert werden, beispielsweise durch Umsetzen mit Verbindungen der allgemeinen Formel XV

(s. Formel XV)

worin $R^{1'}$ obige Bedeutung besitzt und Hal für Chlor, Brom oder Jod steht.

Die Ester der Formel IIIb

(s. Formel IIIb)

worin $R^6$ und Z obige Bedeutung besitzen, und $R^9$ niederes Alkyl bedeutet, können auf an sich bekannte Weise ausgehend von Verbindungen der Formel XVI

(s. Formel XVI)

worin $R^6$ und Z obige Bedeutung besitzen, erhalten werden, indem man die Verbindungen der Formel XVI durch Behandeln mit Natriumnitrit in die entsprechenden N-Nitroso-Verbindungen überführt und diese zu den Hydrazin-Verbindungen der allgemeinen Formel XVII

(s. Formel XVII)

worin $R^6$ und Z obige Bedeutung besitzen, reduziert und die Hydrazin-Verbindungen der Formel XVII weiter mit Brenztraubensäure-niederalkylestern der allgemeinen Formel XVIII

(s. Formel XVIII)

worin $R^9$ obige Bedeutung besitzt, auf an sich bekannte Weise unter den Bedingungen der Fischerschen Indolsynthese umsetzt, wobei intermediär Hydrazon-Verbindungen der Formel XIX

(s. Formel XIX)

worin $R^6$, $R^9$ und Z obige Bedeutung besitzen, gebildet werden, welche weiter zu den Estern der Formel IIIb kondensieren. Für die Reduktion der Nitrosoverbindungen können als Reduktionsmittel beispielsweise Lithiumaluminiumhydrid in Tetrahydrofuran oder metallisches Zinkpulver in Gegenwart von Säure eingesetzt werden. Auch eine katalytische Hydrierung der Nitrosoverbindungen zu den Hydrazinen der Formel XVII ist möglich. Vorteilhaft kann die Herstellung der Ester der Formel IIIb ausgehend von den Verbindungen der Formel XVI in einem Eintopf-Verfahren, ohne die einzelnen Zwischenstufen zu isolieren, durchgeführt werden. Hierbei wird das nach Reduktion der N-Nitrosoverbindung erhaltene, die Hydrazinverbindung der Formel XVII enthaltende, zinksalzhaltige Reaktionsgemisch durch Zugabe von Salzsäure weiter angesäuert, und dann wird der Reaktionsmischung Brenztraubensäureester der Formel XVIII zugegeben. Bei Zugabe des Brenztraubensäureesters der Formel XVIII zu dem Reaktionsgemisch entsteht intermediär die Hydrazonverbindung der Formel XIX, welche unter den Reaktionsbedingungen weiter zu dem Ester der Formel IIIb kondensiert.

Die Ester der Formel IIIb können auf an sich bekannte Weise zu den entsprechenden Säuren hydrolysiert und/oder in ein reaktives Säurederivat dieser Säuren überführt werden.

Bei der Überführung in ein Säurehalogenid wird gleichzeitig auch ein Halogensubstituent $R^5$ in die Verbindungen eingeführt.

Die erfindungsgemäßen 1,7-anellierten 1H-Indol-2-carbonsäure-N-(1,4-benzodiazepin-3-yl)amid-Derivate und ihre pharmakologisch akzeptablen Säureadditionssalze besitzen wertvolle pharmakologische Eigenschaften, insbesondere CCK-antagonistische Wirkungen und zeichnen sich durch ein neuartiges günstiges Wirkungsprofil aus. So besitzen die erfindungsgemäßen CCK-antagonistisch wirksamen Verbindungen der Formel I eine ausgeprägte die Magenentleerung fördernde Wirkungskomponente sowie auch hemmende Einflüsse auf die CCK-induzierte exokrine Pankreassekretion. Sie besitzen im zur Förderung der Magenentleerung wirksamen Dosisbereich nur ein geringes Maß an die Gallenblasenkontraktion hemmender Nebenwirkung und zeichnen sich durch eine geringe Toxizität und große therapeutische Breite aus. Aufgrund ihres günstigen Wirkungsprofils sind die Verbindungen geeignet zur Behandlung von CCK-bedingten Störungen der Magenentleerung.

CCK umfaßt Peptide verschiedener Kettenlänge und agiert als Hormon sowie als Neuropeptid. Unter den CCK-Peptiden ist das Octapeptid CCK-8 die kleinste Einheit mit dem vollen CCK-Wirkungsspektrum. Die nachfolgenden pharmakologischen Tests wurden deshalb mit CCK-8 durchgeführt.

Beschreibung der Testmethoden

1. Bestimmung des Bindungsvermögens der Testsubstanzen an periphere CCK-Rezeptoren.

Die Affinität der Verbindungen der Formel I an periphere CCK-Rezeptoren wird in vitro an Rattenpankreashomogenat gemessen. Bestimmt wird die Hemmung der Bindung des physiologisch relevanten Octapeptids CCK-8 an periphere CCK-Rezeptoren durch die Testssubstanzen.

Die Rezeptor-Bindungsstudien werden nach einer Modifikation der Methode von van Dijk et al (J. Neuroscience 4 (1984), 1021-1033) durchgeführt, wobei als Proteaseinhibitor Sojabohnentrypsininhibitor (= SBTI) verwendet wird. Als tritium-markiertes CCK-8 wird das $^{3}$[H]-CCK-8, Code TRK 775, der Fa. Amersham Int., spezifische Aktivität 60-90 Ci/mmol eingesetzt.

Ganze Pankreasdrüsen von durch Enthauptung getöteten männlichen Wistar-Ratten mit einem Körpergewicht von 150-300 g werden vom Fettgewebe befreit und in dem 50-fachen Volumen einer eiskalten Testpufferlösung (10 mmol 2-[4-(2-Hydroxyäthyl)-piperazin-1-yl]äthansulfonsäure (= HEPES), 130 mmol Natriumchlorid, 5 mmol Magnesiumchlorid, 0,02 % Bacitracine, 0,01 % SBTI, pH 7,4) mit einem Homogenisator vom Typ Kinematica Polytron 15 sek. lang homogenisiert. Sodann werden die Homogenate 10 min. lang bei 48.000 g zentrifugiert. Diese Waschmethode wird wiederholt. Nach dem letzten Zentrifugieren wird der jeweils in Form eines Pellets erhaltene Rückstand in seinem 500-fachen Volumen der Testpufferlösung suspendiert und sofort für die Messung verwendet.

Für den Bindungsversuch werden 500 µl des Gewebehomogenats mit 50 µl Testpufferlösung oder 50 µl einer Lösung der zu untersuchenden Verbindung und mit 50 µl einer $^{3}$[H]-CCK-8-Lösung (Endkonzentration 0,3 nM) inkubiert. Als nichtspezifische Bindung wird 0,1 µmol CCK-8 angenommen. Die Inkubationsdauer beträgt 90 min. bei 25 °C. Alle Verbindungen werden in mehreren Konzentrationen je 3-mal gemessen.

Als Testsubstanzlösungen werden wäßrige Lösungen eingesetzt, welche durch geeignete Verdünnung von 60 x $10^{-4}$-molaren wäßrigen Stammlösungen hergestellt werden. In Wasser schwer lösliche Testsubstanzen werden zunächst in 96 %-igem Äthanol gelöst und diese Lösung mit soviel Wasser verdünnt, daß in der zu testenden Lösung die Äthanolkonzentration 1,6 Vol.-% nicht übersteigt.

Die Trennung von gebundenem und freiem $^{3}$[H]-CCK-8 wird durch Filtration über Glasfaserfilter durchgeführt. Nach zweimaligem Waschen mit je 3 ml HEPES-Lösung werden die Filter über Nacht im Dunkeln in der Scintillationslösung (Scintillationslösung SAVE der Fa. Packard) gelegt und in einem Flüssig-Scintillationszähler der Fa. Packard (Typ tri-carb 1500 C) ausgezählt. Es wird als $IC_{50}$ der jeweiligen Testsubstanz diejenige Konzentration bestimmt, welche eine 50 %-ige Hemmung der spezifischen Bindung des tritiierten CCK-8 an die Rezeptoren bewirkt. Aus dieser wird nach der Cheng-Prusoff-Gleichung der entsprechende pki-Wert berechnet.

Die nachfolgende Tabelle A gibt nach der vorstehend beschriebenen Testmethode erhaltene pKi-Werte für die Affinität der Testsubstanzen zu peripheren CCK-Rezeptoren wieder. Die für die Verbindungen der Formel I angegebenen Beispielsnummern beziehen sich auf die nachstehenden Herstellungsbeispiele.

2. Bestimmung der minimalen toxischen Dosis.

Männlichen Mäusen von 20-25 g Gewicht werden per os Maximaldosen von 300 mg/kg der Testsubstanz verabreicht. Die Tiere werden 3 Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder stark toxische Symptome beobachtet werden, werden weiteren Mäusen zunehmend geringere Dosen verabreicht. Die niedrigste Dosis, welche Tod oder starke toxische Symptome hervorruft, wird als minimale toxische Dosis in der nachstehenden Tabelle A angegeben.

Tabelle A

| Bsp. Nr. | in vitro Bindung an peripheren CCK-Rezeptoren (Pankreas) pKi-Werte | Minimale toxische Dosis mg/kg Maus p.o. |
|---|---|---|
| 1 | 8,87 | > 300 |
| 2a | 9,59 | |
| 2b | 7,71 | |
| 4 | 8,60 | > 300 |
| 5 | 8,84 | > 300 |
| 6 | 8,49 | |
| 8 | 8,04 | |
| 9 | 9,10 | > 300 |
| 10 | 8,75 | 300 |
| 11 | 8,62 | 300 |
| 13 | 9,00 | > 300 |
| 15 | 8,91 | |
| 17 | 8,00 | |
| 18 | 8,93 | > 300 |
| 20 | 9,07 | > 300 |
| 22 | 9,04 | > 300 |
| 23 | 8,00 | > 300 |
| 25 | 7,62 | > 300 |
| 26 | 8,21 | |
| 30 | 8,63 | 300 |
| 33 | 8,43 | > 300 |
| 37 | 9,19 | |
| 38 | 9,01 | |

3. Untersuchung der Wirkung der Testsubstanzen auf CCK-induzierte Magenentleerungsstörungen.

CCK-Gaben führen dazu, daß der transpylorische Transport des Speisebreies aus dem Magen in das Duodenum weitgehend blockiert wird. Untersucht wird die Fähigkeit der Testsubstanzen, diesen Blockierungseffekt des CCK aufzuheben.

Es werden weibliche Mäuse vom NMRI-Stamm mit einem Körpergewicht von 20-25 g in Gruppen von jeweils 10 Tieren verwandt. Nach 24-stündigem Futterentzug (Trinkwasser ad libitum) erhalten die Tiere per os eine Dosis der Testsubstanz suspendiert in einem Volumen von 10 ml/kg Körpergewicht einer 1 %-igen Tylose$^R$-Lösung ( = Methylzellulose) oder in einem Volumen von 10 ml/kg Körpergewicht einer Lösungsvermittlerlösung, welche 5 % Glyzerin, 87 % Polyäthylenglycol 400 und 8 % Wasser enthält. Eine Kontrollgruppe erhält jeweils nur die Tylose$^R$-Lösung oder die den Lösungsvermittler Polyäthylenglycol enthaltende Lösung. 60 Min. später werden den Tieren 80 µg/kg CCK-8 subkutan injiziert. Nach weiteren 5 Min. wird den Tieren je 0,3 ml Kohlebrei (5 % Kohle in 2 %-iger Tyloselösung) per os verabreicht. 5 Min. später erfolgt die Tötung und anschließende Sektion der Mäuse. Es wird untersucht, ob Kohlebrei in das Duodenum vorgedrungen ist.

Während unter Kontrollbedingungen ohne CCK-Gabe bei allen Mäusen Kohlebrei im Duodenum zu finden ist, ist bei den Kontrolltiergruppen mit CCK-Gabe der Transport des Speisebreis in das Duodenum verhindert und bei höchstens 5 % der Tiere sind Spuren Kohlebrei im Duodenum zu finden. Es wird untersucht, bei wieviel % der Tiere nach Gabe der Testsubstanzen der CCK-Effekt aufgehoben und Kohlebrei im Duodenum zu finden ist.

In der nachfolgenden Tabelle B werden die nach der vorstehenden Versuchsbeschreibung erhaltenen Ergebnisse für Dosierungen der Testsubstanzen, welche mindestens bei 40 % der Tiere eine Hemmung des CCK-Effektes aufwiesen, angegeben. Wie aus der Tabelle ersichtlich ist, kann die Wirksamkeit der Substanzen durch Verabreichung in einer als Lösungsvermittler PEG 400 enthaltenden Lösung oftmals wesentlich verstärkt werden.

Tabelle B

| Bsp. Nr. | CCK-antagonistiche Wirkung auf CCK-induzierte Magenentleerungsstörung | |
|---|---|---|
| | Dosis µMol/kg Maus p.o. | % Tiere mit Aufhebung des CCK-Effektes |
| 1 | 0,215 | 50 |
| 2a | 0,1 | 100 |
| 2b | 10 | 70 |
| 4 | 1,0 | 45 |
| 5 | 10 | 75 |
| 6 | 0,681 | 40 |
| 9 | 0,215 | 50 |
| 10 | 0,1 | 40 |
| 13 | 10 | 83 |
| | 1,0* | 100 |
| 15 | 1,0 | 57 |
| | 0,10* | 90 |
| 17 | 1,0* | 50 |
| 20 | 1,0 | 50 |
| 27 | 1,0 | 40 |
| 33 | 1,0 | 50 |

* = in Lösungsvermittler enthaltender Lösung verabreicht.

4. Prüfung der Hemmwirkung der Substanzen auf CCK-induzierte Gallenblasenentleerung.

CCK bewirkt eine Kontraktion der Gallenblasenmuskulatur und damit eine Entleerung der Gallenblase. Dies führt zu einer Gewichtsabnahme der Gallenblase gegenüber nicht mit CCK behandelten Kontrolltieren. CCK-8-Gaben von 0,1 µg/kg intraperitoneal können zu Gewichtsabnahmen bis auf ca. 1/10 des Ursprungsgewichtes der Gallenblasen führen.

Es werden weibliche Mäuse vom NMRI-Stamm mit einem Körpergewicht von 20-25 g in Gruppen von jeweils 10 Tieren verwandt. Nach 24-stündigem Futterentzug (Trinkwasser ad libitum) erhalten die Tiere per os eine Dosis der Testsubstanz suspendiert in einem Volumen von 10 ml/kg Körpergewicht einer 1 %-igen $Tylose^R$-Lösung (= Methylzellulose) oder in einem Volumen von 10 ml/kg Körpergewicht einer Lösungsvermittlerlösung, welche 5 % Glyzerin, 87 % Polyäthylenglycol 400 und 8 % Wasser enthält. Zwei Kontrollgruppen erhalten jeweils nur die $Tylose^R$-Lösung oder die den Lösungsvermittler Polyäthylenglycol enthaltende Lösung. Eine der Kontrollgruppen und alle mit Testsubstanz behandelten Tiere erhalten 60 Min. später 0,1 µg/kg CCK-8 i.p. injiziert.

5 Min. nach der CCK-8-Applikation werden die Mäuse getötet, die Gallenblasen herauspräpariert und gewogen.

In der nachfolgenden Tabelle C wird die durch die Testsubstanzen bewirkte prozentuale Hemmung des CCK-8-Effektes auf die Gallenblasengewichte angegeben.

Tabelle C

| Bsp. Nr. | CCK-antagonistische Wirkung auf Gallenblase | |
|---|---|---|
| | Dosis µMol/kg Maus p.o. | %Hemmung der CCK-induzierten Gallenblasenentleerung |
| 1 | 10 | 66 |
| | 1 | < 25 |
| 2a | 0,46 | 16 |
| 2b | 46 | 8,3 |
| 6 | 10 | 73 |
| 9 | 100 | 99 |
| | 4,6 | < 25 |
| 15 | 100 | 100 |
| | 10 | < 25 |
| | 0,1* | < 25 |
| 20 | 10 | 25 |
| 33 | 10 | 19 |

Ein Vergleich der Daten der Tabellen B und C zeigt, daß CCK-antagonistische Wirkungen der Substanzen an der Gallenblase erst bei Dosen beobachtet werden, welche um ein Vielfaches über den gegen CCK-induzierte Magenentleerungsstörungen wirksamen Dosenbereichen liegen.

Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich jedoch für Applikationen in größeren Säugetieren, insbesondere Menschen, Arzneiformen mit einem Wirkstoffgehalt von 5-50 mg pro Einzeldosis.

Als Heilmittel können die Verbindungen der Formel I und ihre physiologisch verträglichen Säureadditionssalze mit üblichen pharmazeutischen Hilfsstoffen in galenischen Zubereitungen, wie z.B. Tabletten, Kapseln, Suppositorien oder Lösungen enthalten sein. Die Verbindungen der Formel I zeichnen dich durch eine gute Löslichkeit in pharmazeutisch übliche Hilfsstoffe enthaltenden Lösungen und eine hohe Resorbierbarkeit aus. Die galenischen Zubereitungen können nach an sich bekannten Methoden hergestellt werden unter Verwendung üblicher fester Trägerstoffe wie z.B. Milchzucker, Starke oder Talkum oder flüssiger Verdünnungsmittel wie z.B. Wasser, fetten Ölen oder flüssigen Parafffinen und unter Verwendung von pharmazeutisch üblichen Hilfsstoffen, beispielsweise Tablettensprengmitteln oder Konservierungsmitteln.

Die nachfolgenden Beispiele sollen die Erfindung näher erläutern.

Beispiel 1:

3-[(4H-Pyrrolo[3,2,1-ij]-5,6-dihydrochinolin-2-carbonyl)-amino]-8-methoxy-1-methyl-2-oxo-5-phenyl-1H-2,3-dihydro-1,4-benzodiazepin.

A) 43 g $N_1$-Methyl-$N_1$-(3-methoxyphenyl)-2-hydroxy-1,3-diaminopropan und 31 ml Triäthylamin wurden in 280 ml Dichlormethan gelöst. Unter Eiskühlung wurde in die Lösung langsam eine Lösung von 24,4 ml Benzoylchlorid in 20 ml Dichlormethan getropft. Das Reaktionsgemisch wurde 1,5 Stunden bei Raumtemperatur gerührt. Anschließend wurde die Reaktionslösung zur Aufarbeitung mit Wasser und mit Natriumchloridlösung gewaschen und das Lösungsmittel abgedampft. Als Rückstand verblieben 70,0 g rohes $N_1$-Methyl-$N_1$-(3-methoxyphenyl)-$N_2$-benzoyl-2-hydroxy-1,3-diaminopropan. Das nach Umkristallisation aus Toluol/Isopropanol erhaltene reine Produkt hatte einen Schmelzpunkt von 87-89 °C.

B) 64 g des vorstehend erhaltenen Produktes wurden in 64 ml Phosphoroxidtrichlorid 1 1/2 Stunden im Ölbad bei 130 °C Badtemperatur reagieren lassen. Anschließend wurde abgekühlt, mit Dichlormethan verdünnt und die Lösung mit Eiswasser versetzt. Die organische Phase wurde abgetrennt, mehrmals mit Wasser gewaschen, dann mit verdünnter Natriumhydroxidlösung behandelt, wiederum mit Wasser gewaschen, über Natriumsulfat getrocknet und eingedampft. Als Rückstand wurden 56,7 g eines öligen Rohproduktes erhalten, welches ein Gemisch aus ca. 60 % 2-Chlormethyl-1-methyl-8-methoxy-5-phenyl-2,3-dihydro-1H-1,4-benzodiazepin und ca. 40 % 3-Chlor-1-methyl-9-methoxy-6-phenyl-1,2,3,4-tetrahydro-1,5-benzodiazocin enthielt. Zur Isomerisierung des Benzodiazozins-Anteils wurde das rohe Gemisch in

222 ml Tetrachloräthan 30 Minuten unter Rückfluß erhitzt. Anschließend wurde das Tetrachloräthan abgedampft und das verbleibende 2-Chlormethyl-1-methyl-8-methoxy-5-phenyl-2,3-dihydro-1H-1,4-benzodiazepin wurde ohne Reinigung in der nächsten Reaktionsstufe weiterverarbeitet.

C) 56,7 g des vorstehend erhaltenen Produktes wurden in 285 ml Dichlormethan gelöst. Zu der Lösung wurden 322 ml 32 %-ige wäßrige Salzsäure, 2481 ml Wasser und 255 ml Dichlormethan zugegeben. Anschließend wurde eine Lösung von 32,4 g Kaliumpermanganat in 660 ml Wasser zugetropft, wobei durch Eiskühlung die Innentemperatur auf unter 15 °C gehalten wurde. Sodann wurde das Reaktionsgemisch noch eine halbe Stunde bei Raumtemperatur gerührt. Zur Aufarbeitung wurde dem Reaktionsgemisch festes Natriumbicarbonat portionsweise bis zur Neutralisierung zugesetzt. Dann wurde die Lösung von dem gebildeten Niederschlag über Asbestschlamm (Handelsprodukt Theorit$^{R}$) abgesaugt, die Dichlormethanphase abgetrennt und die wäßrige Phase nochmals mit Dichlormethan extrahiert. Die vereinigten Dichlormethanextrakte wurden mit verdünnter Natronlauge und anschließend mit Wasser gewaschen, über Magnesiumsulfat getrocknet und eingedampft. Es wurden 55 g Rohprodukt erhalten. Aus dem Rohprodukt wurde mittels Säulenchromatographie an Kieselgel unter leicht erhöhtem Druck (Flash-Chromatographie) unter Verwendung von Cyclohexan/Essigsäureäthylester 4 : 6 als Elutionsmittel das reine 1-Methyl-8-methoxy-2-oxo-5-phenyl-2,3-dihydro-1H-1,4-benzodiazepin isoliert. Es wurden 15,5 g öliges Reinprodukt erhalten.

D) 15,5 g des vorstehenden Produktes wurden in 293 ml Toluol suspendiert. Die Suspension wurde auf -20 °C abgekühlt und sodann wurden 16,4 g Kalium-tert.-Butylat unter Rühren zugegeben und das Gemisch noch weitere 15 Minuten gerührt. Dann wurden 9,4 ml Isoamylnitrit so langsam unter Kühlung zugegeben, daß die Temperatur des Reaktionsgemisches unter 0 °C blieb. Anschließend wurde noch 30 Minuten bei 0 °C gerührt. Sodann wurde die Reaktionslösung zur Aufarbeitung unter Rühren in eine Mischung aus 586 ml eiskaltem Wasser, 29 ml Eisessig und 586 ml Essigsäureäthylester gegeben. Es wurde kräftig durchgemischt und sodann die organische Phase abgetrennt und die wäßrige Phase nochmals mit Essigsäureäthylester extrahiert. Die vereinigten organischen Phasen wurden mit Wasser gewaschen und eingedampft. Es wurden 21 g Rohprodukt erhalten. Dieses wurde aus Toluol/Äthanol kristallisiert. Das Kristallisat wurde abgetrennt und die Mutterlauge wurde nochmals durch Flash-Chromatographie über 100 g Kieselgel unter Verwendung von Cyclohexan/Essigsäureäthylester 4 : 6 als Elutionsmittel gereinigt und eingedampft. Der Rückstand wurde aus Äthanol kristallisiert und mit dem obigen Kristallisat vereinigt. Insgesamt wurden 9,5 g 3-Hydroxyimino-1-methyl-8-methoxy-2-oxo-5-phenyl-2,3-dihydro-1H-1,4-benzodiazepin mit einem Schmelzpunkt von 206-207 °C erhalten.

E) 9,5 g des vorstehend erhaltenen Produktes wurden in ein Gemisch aus 700 ml Eisessig und 80 ml Trifluoressigsäure gegeben. Das Reaktionsgemisch wurde auf 40 °C (Innentemperatur) erwärmt und es wurden insgesamt 6,9 g Zinkstaub portionsweise unter Rühren zugegeben, das Gemisch weitere 2 Stunden bei 40 °C gerührt und sodann nochmals 1 g Zinkstaub zuggeben und weitere 1 1/2 Stunden bei 40 °C gerührt. Zur Aufarbeitung wurde das Gemisch mit Toluol verdünnt, abkühlen gelassen und eingedampft. Der verbleibende Rückstand wurde in Dichlormethan aufgenommen, mit wäßriger Natriumcarbonatlösung und Wasser gewaschen. Feststoffe wurden über Theorit$^{R}$ abgesaugt, die Lösung getrocknet und eingedampft. Es wurden 8,1 g rohes 3-Amino-1-methyl-8-methoxy-2-oxo-5-phenyl-2,3-dihydro-1H-1,4-benzodiazepin erhalten, welches ohne Reinigung in der nachfolgenden Reaktionsstufe H weiterverarbeitet wurde.

F) 150 g 1,2,3,4-Tetrahydrochinolin wurden in 1,25 l Eisessig gelöst. Zu der Lösung wurde unter Kühlung im Eisbad auf ca. 15 °C Innentemperatur eine Lösung von 80 g Natriumnitrit in 300 ml Wasser gegeben und das Reaktionsgemisch wurde 45 Minuten lang weiter gerührt. Zu der das gebildete N-Nitroso-1,2,3,4-Tetrahydrochinolin enthaltenden Reaktionslösung wurden während 1,5 Stunden portionsweise 300 g Zinkstaub zugegeben, wobei das Reaktionsgemisch durch Kühlen im Eisbad auf einer Innentemperatur von 15-20 °C gehalten wurde. Anschließend wurden 1,75 l Wasser und 1,25 l 32 %-ige wäßrige Salzsäure zu dem Gemisch gegeben und weitere 1,5 Stunden gerührt. Zu dem das gebildete N-Amino-1,2,3,4-tetrahydrochinolin und das Zinksalz enthaltenden sauren Reaktionsgemisch wurden 130 g Brenztraubensäureäthylester gegeben und das Gemisch 1 1/2 Stunden am Rückfluß erhitzt und anschließend weitere 16 Stunden stehen gelassen. Hierbei wurde das intermediär gebildete Hydrazon in situ zu 4H-Pyrrolo[3,2,1-ij]-5,6-dihydrochinolin-2-carbonsäureäthylester kondensiert. Zur Aufarbeitung wurde das Reaktionsgemisch zweimal mit insgesamt 5 l Dichlormethan extrahiert, die Dichlormethanextrakte vereinigt, zweimal mit insgesamt 1 l Wasser gewaschen, über Natriumsulfat getrocknet und eingeengt. Es wurden 280 g roher 4H-Pyrrolo[3,2,1-ij]-5,6-dihydrochinolin-2-carbonsäureäthylester erhalten, welche durch Chromatographie an Kieselgel unter Verwendung von Dichlormethan als Eluens gereinigt wurden. Es wurden 151,8 g gereinigtes Produkt mit einem Schmelzpunkt von 70-72 °C erhalten.

G) 39 g 4H-Pyrrolo[3,2,1-ij]-5,6-dihydrochinolin-2-carbonsäureäthylester wurden in 40 ml Äthanol gelöst und diese Lösung wurde bei Raumtemperatur zu einer Lösung von 11,3 g Kaliumhydroxid in einem Gemisch aus 20 ml Wasser und 145 ml Äthanol gegeben. Das Reaktionsgemisch wurde 90 min. bei Raumtemperatur gerührt und dann auf 10 °C abgekühlt. Der ausgefallene Feststoff wurde abgesaugt und dreimal mit je 30 ml Äthanol gewaschen. Die Mutterlauge wurde auf die Hälfte eingeengt, und der dabei ausfallende Feststoff wurde ebenfalls abgetrennt und mit Äthanol gewaschen.

Der gesamte Feststoff wurde nun in 150 ml Wasser gelöst und daraus die Säure durch Ansäuern der Lösung mit konzentrierter Salzsäure auf pH 1 bis 2 gefällt. Die ausgefallene Säure wird abgetrennt, dreimal mit je 40 ml Wasser gewaschen und bei 60 °C getrocknet. Es wurden 32,4 g 4H-Pyrrolo[3,2,1-ij]-5,6-dihydrochinolin-2-carbonsäure mit einem Schmelzpunkt von 212-213 °C (Z) erhalten.

H) 5,4 g 4H-Pyrrolo[3,2,1-ij]-5,6-dihydrochinolin-2-carbonsäure und 3,67 ml Triäthylamin wurden in 119 ml Dichlormethan gelöst. Die Lösung wurde auf -20 °C abgekühlt und es wurden langsam unter Rühren 2,08 ml Methansulfonsäurechlorid zugetropft und das Reaktionsgemisch 15 Minuten bei -20 °C weitergerührt. Zu der das gemischte Anhydrid aus Methansulfonsäure und der 4H-Pyrrolo[3,2,1-ij]-5,6-dihydrochinolin-2-carbonsäure enthaltenden Reaktionslösung wurde unter Rühren eine Lösung von 8 g des in Stufe E erhaltenen 3-Amino-1-methyl-8-methoxy-2-oxo-5-phenyl-2,3-dihydro-1H-1,4-benzodiazepin und 3,67 ml Triäthylamin in 100 ml Dichlormethan bei einer Temperatur zwischen -15 und -20 °C zugetropft, das Reaktionsgemisch weitere 30 Minuten bei -15 °C gerührt und langsam (innerhalb einer Stunde) auf Raumtemperatur erwärmen lassen. Zur Aufarbeitung wurde das Reaktionsgemisch mit Wasser verdünnt, die Dichlormethanphase abgetrennt, mit Natriumbicarbonatlösung und anschließend mit Wasser gewaschen, über Natriumsulfat getrocknet, filtriert und eingedampft. Als Rückstand wurden 14,3 g der rohen Titelverbindung erhalten. Das Rohprodukt wurde mittels Säulenchromatographie an 700 g Kieselgel unter leicht erhöhtem Druck (Flash-Chromatographie) unter Verwendung von Cyclohexan/Essigsäureäthylester 1 : 1 als Elutionsmittel gereinigt. Es wurden 6,1 g gereinigtes Produkt erhalten. Dieses wurde aus 25 ml Äthanol mit einem geringen Zusatz von Dichlormethan kristallisiert und die Kristalle wurden 2 Tage bei 80 °C getrocknet. Es wurden 3,3 g racemisches 3-[(4H-Pyrrolo[3,2,1-ij]-5,6-dihydrochinolin-2-carbonyl)-amino]-8-methoxy-1-methyl-2-oxo-5-phenyl-1H-2,3-dihydro-1,4-benzodiazepin mit einem Schmelzpunkt von 175-178 °C erhalten.

Beispiel 2:

Herstellung der optischen Isomeren von 3-[(4H-Pyrrolo[3,2,1-ij]-5,6-dihydrochinolin-2-carbonyl)-amino]-8-methoxy-1-methyl-2-oxo-5-phenyl-1H-2,3-dihydro-1,4-benzodiazepin.

2a: (-)-Isomeres, optischer Drehwert $[\alpha]_D^{20}$ = -88,2° (c = 0,5 in Dichlormethan).

A) 30,5 g racemisches 3-Amino-1-methyl-8-methoxy-2-oxo-5-phenyl-2,3-dihydro-1H-1,4-benzodiazepin (hergestellt analog Beispiel 1 E) wurden in 190 ml Dimethylformamid gelöst. Zu der Lösung wurden unter Feuchtigkeitsausschluß nacheinander 28,8 g N-tert.Butoxycarbonyl-D-phenylalanin (= BOC-D-Phenylalanin), 15 g 1-Hydroxybenzotriazol, 20,7 g 1-Äthyl-3-[3-(dimethylamino)-propyl]-carbodiimid-hydrochlorid und 15 ml Triäthylamin gegeben. Das Reaktionsgemisch wurde 30 min. bei Raumtemperatur gerührt. Zur Aufarbeitung wurde das Dimethylformamid unter vermindertem Druck abdestilliert und der Rückstand in Essigsäureäthylester gelöst. Die Lösung wurde mit 10 %-iger wäßriger Zitronensäurelösung geschüttelt, die Phasen voneinander getrennt, die wäßrige Phase nochmals mit Essigsäureäthylester extrahiert und anschließend die vereinigten organischen Phasen mit 10 %-iger wäßriger Natronlauge, Wasser und wäßriger Kochsalzlösung gewaschen, über Natriumsulfat getrocknet und filtriert. Nach dem Abdestillieren des Lösungsmittels wurden 66 g Rohprodukt erhalten, welches nochmals durch Flash-Chromatographie über 700 g Kieselgel unter Verwendung von Cyclohexan/Essigsäureäthylester 1 : 1 als Elutionsmittel gereinigt wurde. Nach Eindampfen des Lösungsmittels wurden 60 g 1,1-Dimethyläthyl-N-{-(R)-2-[(2,3-dihydro-1-methyl-2-oxo-5-phenyl-8-methoxy-1H-1,4-benzodiazepin-3-yl)-amino]-2-oxo-1-benzyläthyl}-carbamat als ein 1 : 1 Distereomerengemisch erhalten.

B) 60 g des vorstehend erhaltenen diastereomeren Amidgemisches wurden in 480 ml Essigsäureäthylester gelöst. Zur Abspaltung der BOC-Schutzgruppe aus den Amiden wurde die Lösung mit gasförmigem Chlorwasserstoff gesättigt und das Rektionsgemisch 30 min. gerührt. Hierbei fiel ein Kristallisat von Hydrochlorid der freigesetzten Amine an, in welchem das (-)-drehende Diastereomere angereichert war. Das Kristallisat wurde abgesaugt. Dreimaliges Umkristallisieren aus Äthanol ergab ein diastereomerenreines Hydrochlorid des in Dichlormethanlösung (-)-drehenden 3-Phenyl-2-amino-N-(2,3-dihydro-1-methyl-2-oxo-5-phenyl-8-methoxy-1H-1,4-benzodizepin-3-yl)-propionsäureamids. Zur Freisetzung der Base wurde das Hydrochlorid mit 10 %-iger Natronlauge versetzt und die Base mit Essigsäureäthylester aus der wäßrigen Phase extrahiert. Nach Waschen, Trocknen und Eindampfen der organischen Phase wurden 18,4 g diastereomerenreines 3-Phenyl-2-amino-N-(2,3-dihydro-1-methyl-2-oxo-5-phenyl-8-methoxy-1H-1,4-benzodiazepin-3-yl)-propionsäureamid erhalten. Drehwert $[\alpha]_D^{20}$ von -30,6° (c = 0,5 in Dichlormethan)

C) 18,4 g des vorstehend erhaltenen in Dichlormethanlösung (-)-drehenden diastereomerenreinen Amids wurden in 100 ml Dichlormethan gelöst. Unter Feuchtigkeitsausschluß wurden zu der Lösung 5,4 ml Phenylisothiocyanat gegeben und das Reaktionsgemisch wurde 10 min. bei Raumtemperatur gerührt. Dann wurde das Dichlormethan unter vermindertem Druck abdestilliert, und der verbleibende Rückstand wurde durch Flash-Chromatographie über 500 g Kieselgel unter Verwendung von Cyclohexan/Essigsäureäthylester 1 : 1 als Elutionsmittel gereinigt. Nach Eindampfen des Lösungsmittels wurde ein Schaumharz erhalten, welches aus Äthanol kristallisiert wurde. Es wurden 20,1 g $N_1$-Phenyl-$N_2$-{2-[-(2,3-dihydro-1-methyl-2-oxo-5-phenyl-8-methoxy-1H-1,4-benzodiazepin-3-yl)-amino]-2-oxo-1-(benzyl)-äthyl}-thioharnstoff mit einem Schmelzpunkt von 138-160 °C erhalten. Optischer Drehwert: $[\alpha]_D^{20}$ = - 11,2° (c = 0,5 in Methanol).

D) 20 g des vorstehend erhaltenen Thioharnstoff-Produktes wurden mit 30,7 ml Trifluoressigsäure versetzt, und das Reaktionsgemisch 20 min. auf 50 °C erwärmt. Dann wurde die Trifluoressigsäure unter vermindertem Druck abdestilliert, der verbleibende Rückstand zweimal mit Dichlormethan abgeraucht, erneut in Dichlormethan gelöst und zur Reinigung an 500 g Kieselgel flash-chromatographiert, wobei als Laufmittel zunächst ein Gemisch aus Cyclohexan/Essigsäureäthylester 1 : 1 und dann ein Gemisch aus Dichlormethan/Methanol/Essigsäure/Wasser 90 : 10 : 1 : 1 verwendet wurden. Das erhaltene Hydrotrifluoroacetat des (-)-3-Amino-2,3-dihydro-1-methyl-2-oxo-5-phenyl-8-methoxy-1H-1,4-benzodiazepin wurde in Dichlormethan gelöst, die Lösung zur Freisetzung des Amins mit wäßriger Natriumbicarbonatlösung versetzt und das Reaktionsgemisch mit Dichlormethan extrahiert. Die Dichlormethanphase wurde abgetrennt, über Natriumsulfat getrocknet, filtriert, das Lösungsmittel unter vermindertem Druck abdestilliert und die zurückbleibende Base getrocknet. Es wurden 8,5 g schaumiges (-)-3-Amino-2,3-dihydro-1-methyl-2-oxo-5-phenyl-8-methoxy-1H-1,4-benzodiazepin erhalten. Optischer Drehwert $[\alpha]_D^{20}$ = -171,2° (c = 0,5 in Dichlormethan).

E) Analog der in Beispiel 1 H) beschriebenen Methode wurden 7,0 g des vorstehend erhaltenen (-)-3-Amino-1-methyl-8-methoxy-2-oxo-5-phenyl-2,3-dihydro-1H-1,4-benzodiazepin mit einer das gemischte Anhydrid aus 4,9 g 4H-Pyrrolo[3,2,1-ij]-5,6-dihydrochinolin-2-carbonsäure und 1,85 ml Methansulfonsäurechlorid in Dichlormethan enthaltenden Reaktionslösung umgesetzt. Das Reaktionsgemisch wurde wie in Beispiel 1 H) beschrieben aufgearbeitet. Nach flash-chromatographischer Reinigung wurden 9,0 g gereinigtes kristallines Produkt erhalten. Dieses wurde zur Entfernung etwaiger enantiomerer Verunreinigungen zwei weitere Male aus Methanol und einmal aus Äthanol umkristallisiert. Es wurden 6,7 g enantiomerenreines (-)-3-[(4H-Pyrrolo[3,2,1-ij]-5,6-dihydrochinolin-2-carbonyl)-amino]-8-methoxy-1-methyl-2-oxo-5-phenyl-1H-2,3-dihydro-1,4-benzodiazepin mit einem Schmelzpunkt von 201-205 °C und einem optischen Drehwert $[\alpha]_D^{20}$ von -88,2° (c = 0,5 in Dichlormethan) erhalten.

2 b: In Dichlormethan (+)-drehendes 3-[(4H-Pyrrolo[3,2,1-ij]-5,6-dihydrochinolin-2-carbonyl)-amino]-8-methoxy-1-methyl-2-oxo-5-phenyl-1H-2,3-dihydro-1,4-benzodiazepin.

A) Die bei der Herstellung des diastereomerenreinen Hydrochlorids unter 2a B) anfallenden Mutterlaugen wurden zur Gewinnung des anderen diastereomeren Hydrochlorids eingedampft. Das ausfallende Hydrochlorid wurde 4 mal aus einem Gemisch aus Acetonitril und Isopropylacetat umkristallisiert. Aus dem Hydrochlorid wurden analog der vorstehend beschriebenen Methode 14,5 g diastereomerenreines in methanolischer Lösung (+)-drehenden 3-Phenyl-2-amino-N-(2,3-dihydro-1-methyl-2-oxo-5-phenyl-8-methoxy-1H-1,4-benzodiazepin-3-yl)-propionsäureamid mit einem optischen Drehwert $[\alpha]_D^{20}$ = + 162,6° (c = 0,5 in Methanol) erhalten.

B) 14,5 g des vorstehend erhaltenen in methanolischer Lösung (+)-drehenden Amids wurden analog Beispiel 2a C) mit 4,3 ml Phenylisothiocyanat in Dichlormethan umgesetzt. Das Reaktionsgemisch wurde wie unter Beispiel 2a C) beschrieben aufgearbeitet. Es wurden 18,6 g $N_1$-Phenyl-$N_2${2-[(2,3-dihydro-1-methyl-2-oxo-5-phenyl-8-methoxy-1H-1,4-benzodiazepin-3-yl)-amino]-2-oxo-1-(benzyl)-äthyl}-thioharnstoff erhalten. Optischer Drehwert $[\alpha]_D^{20}$ = +60,2° (c = 0,5 in Methanol).

C) 18,5 g des vorstehend erhaltenen Thioharnstoff-Produktes wurden mit 28,4 ml Trifluoressigsäure analog Beispiel 2a D) umgesetzt. Aus dem erhaltenen Hydrotrifluoroacetat des (+)-3-Amino-2,3-dihydro-1-methyl-2-oxo-5-phenyl-8-methoxy-1H-1,4-benzodiazepin wurde das Amin analog Beispiel 2a D) freigesetzt. Es wurden 7,7 g (+)-3-Amino-2,3-dihydro-1-methyl-2-oxo-5-phenyl-8-methoxy-1H-1,4-benzodiazepin erhalten. Optischer Drehwert
$[\alpha]^{20}{}_{D}$ = +143,8° (c = 0,5 in Dichlormethan).

D) Analog der in Beispiel 1 H) beschriebenen Methode wurden 5,0 g des vorstehend erhaltenen (+)-3-Amino-1-methyl-8-methoxy-2-oxo-5-phenyl-2,3-dihydro-1H-1,4-benzodiazepin mit einer das gemischte Anhydrid aus 3,49 g 3-[(4H-Pyrrolo[3,2,1-ij]-5,6-dihydrochinolin-2-carbonsäure und 1,32 ml Methansulfonsäurechlorid in Dichlormethan enthaltenden Reaktionslösung umgesetzt. Das Reaktionsgemisch wurde wie in Beispiel 1 H) beschrieben aufgearbeitet. Es wurden 10 g Rohprodukt erhalten. Nach flash-chromatographischer Reinigung wurde zur Entfernung etwaiger enantiomerer Verunreinigungen dreimal aus Methanol umkristallisiert. Es wurden 3,4 g enantiomerenreines (+)-3-[(4H-Pyrrolo[3,2,1-ij]-5,6-dihydrochinolin-2-carbonyl)-amino]-8-methoxy-1-methyl-2-oxo-5-phenyl-1H-2,3-dihydro-1,4-benzodiazepin mit einem Schmelzpunkt von 201-205 °C erhalten. Optischer Drehwert
$[\alpha]^{20}_{D}$ = +88,4° (c = 0,5 in Dichlormethan).

Beispiel 3:

3-[(4H-Pyrrolo[3,2,1-ij]-5,6-dihydrochinolin-2-carbonyl)-amino]-1-n-pentyl-2-oxo-1H-2,3-dihydro-1,4-benzodiazepin.

A) 10 g 2-Oxo-5-phenyl-2,3-dihydro-1H-1,4-benzodiazepin wurden in 100 ml Tetrahydrofuran unter Stickstoffatmosphäre gelöst. Zu der Lösung wurden unter Stickstoffatmosphäre portionsweise 1,4 g Natriumhydrid in Form einer 80 %-igen öligen Suspension zugegeben und das Reaktionsgemisch 30 Minuten am Rückfluß erhitzt. Es wurden langsam 9,2 g (=5,5 ml) Jodpentan zugetropft, die Mischung weitere 1,5 Stunden am Rückfluß erhitzt und dann nochmals 0,3 g Natriumhydrid in Form einer öligen Suspension zugegeben, und nach weiteren 10 Minuten nochmals 5,5 ml Jodpentan zugetropft und das Gemisch eine weitere Stunde am Rückfluß erhitzt. Zur Aufarbeitung wurde das Reaktionsgemisch mit Eiswasser versetzt, mit Dichlormethan verdünnt, die wäßrige Phase abgetrennt, die organische Phase mit Wasser neutral gewaschen, über Natriumsulfat getrocknet, filtriert und eingedampft. Es wurden 13,5 g Rohprodukt erhalten, welches durch Flash-Chromatographie über ca. 300 g Kieselgel unter Verwendung von Cyclohexan/Essigsäureäthylester 1 : 1 als Elutionsmittel gereinigt wurde. Das gereinigte Produkt wurde aus Cyclohexan kristallisiert und getrocknet. Es wurden 6,2 g reines 2-Oxo-1-n-pentyl-5-phenyl-2,3-dihydro-1H-1,4-benzodiazepin mit einem Schmelzpunkt von 93-95 °C erhalten.

B) 6,5 g des vorstehenden Produktes wurden in 122 ml Toluol suspendiert. Die Suspension wurde auf -20 °C abgekühlt und sodann wurden 5,88 g Kalium-tert.-butylat unter Rühren zugegeben und das Gemisch wie in Beispiel 1D beschrieben mit 2,84 ml tert. Butylnitrit umgesetzt. Das Reaktionsgemisch wurde wie in Beispiel 1D aufgearbeitet. Hierbei wurden 4,7 g 3-Hydroxyimino-2-oxo-1-n-pentyl-5-phenyl-2,3-dihydro-1H-1,4-benzodiazepin mit einem Schmelzpunkt von 188-191 °C erhalten.

C) 4,6 g des vorstehend erhaltenen Produktes wurden in einem Gemisch aus 328 ml Eisessig und 37,6 ml Trifluoressigsäure gelöst und wie in Beispiel 1E beschrieben mit insgesamt 3,1 g Zinkstaub reduziert. Das Reaktionsgemisch wurde wie in Beispiel 1E aufgearbeitet. Es wurden 4,2 g rohes 3-Amino-2-oxo-1-n-pentyl-5-phenyl-2,3-dihydro-1H-1,4-benzodiazepin erhalten, welches ohne Reinigung weiterverarbeitet wurde.

D) Zu einer das gemischte Anhydrid aus Methansulfonsäure und 2,6 g 4H-Pyrrolo-[3,2,1-ij]-5,6-dihydrochinolin-2-carbonsäure in 60 ml Dichlormethan enthaltenden Reaktionslösung wurde nach dem in Beispiel 1H) beschriebenen Verfahren eine Lösung von 4,2 g des vorstehend erhaltenen Produktes und 1,77 ml Triäthylamin in 50 ml Dichlormethan bei einer Temperatur zwischen -15 °C und -20 °C zugetropft. Das Reaktionsgemisch wurde wie in Beispiel 1H) beschrieben aufgearbeitet. Es wurden 3,9 g 3-[(4H-Pyrrolo[3,2,1-ij]-5,6-dihydrochinolin-2-carbonyl)-amino]-2-oxo-1-n-pentyl-5-phenyl-1H-2,3-dihydro-1,4-benzodiazepin als weißes Schaumharz erhalten.
IR-Spektrum: 1682 $cm^{-1}$, 1662 $cm^{-1}$, 1524 $cm^{-1}$, 1499 $cm^{-1}$.

Beispiel 4:

3-[(4H-Pyrrolo[3,2,1-ij]-5,6-dihydrochinolin-2-carbonyl)-amino]-1-methyl-2-oxo-5-cyclohexyl-1H-2,3-dihydro-1,4-benzodiazepin.

A) 75,6 g $N_1$-Methyl-$N_1$-phenyl-2-hydroxy-1,3-diaminopropan und 65 ml Triäthylamin wurden in 600 ml Dichlormethan gelöst. Unter Eiskühlung wurde in die Lösung langsam eine Lösung von 61 ml Cyclohexylcarbonsäurechlorid in 50 ml Dichlormethan zugetropft. Das Reaktionsgemisch wurde 1,5 Stunden bei Raumtemperatur gerührt. Anschließend wurde die Reaktionslösung zur Aufarbeitung mit Wasser und mit Natriumchloridlösung gewaschen und das Lösungsmittel abgedampft. Als Rückstand verblieben 131 g rohes $N_1$-Methyl-$N_1$-phenyl-$N_2$-cyclohexylcarbonyl-2-hydroxy-1,3-diaminopropan. Das Rohprodukt wurde aus Toluol umkristallisiert, mit Ether gewaschen und getrocknet. Es wurden 113,8 g reines Produkt mit einem Schmelzpunkt von 86 - 88 °C erhalten.

B) 87 g des vorstehend erhaltenen Produktes wurden in 174 ml Phosphoroxidtrichlorid 2 Stunden im Ölbad bei 130 °C Badtemperatur reagieren lassen. Anschließend wurde abgekühlt, mit Dichlormethan verdünnt und die Lösung mit Eiswasser versetzt.Die organische Phase wurde abgetrennt, mehrmals mit Wasser gewaschen, dann mit verdünnter Natriumhydroxidlösung behandelt, wiederum mit Wasser gewaschen, über Natriumsulfat getrocknet und eingedampft. Als Rückstand wurden 76,1 g eines öligen Rohproduktes erhalten, welches ein Gemisch aus ca. 40 % 2-Chlormethyl-1-methyl-5-cyclohexyl-2,3-dihydro-1H-1,4-benzodiazepin und ca. 60 % 3-Chlor-1-methyl-6-cyclohexyl-1,2,3,4-tetrahydro-1,5-benzodiazocin enthielt. Zur Isomerisierung des Benzodiazozin-Anteils wurde das rohe Gemisch in 300 ml Tetrachloräthan 30 Minuten unter Rückfluß erhitzt. Anschließend wurde das Tetrachloräthan abgedampft, und das verbleibende 2-Chlormethyl-1-methyl-5-cyclohexyl-2,3-dihydro-1H-benzodiazepin wurde ohne Reinigung in der nächsten Reaktionsstufe weiterverarbeitet.

C) 19,0 g des vorstehend erhaltenen Produktes wurden in 103 ml Dichlormethan gelöst. Zu der Lösung wurden 116 ml 32 %-ige wäßrige Salzsäure, 882 ml Wasser und 91 ml Dichlormethan zugegeben. Anschließend wurde eine Lösung von 11,65 g Kaliumpermanganat in 238 ml Wasser zugetropft, wobei durch Eiskühlung die Innentemperatur auf unter 15 °C gehalten wurde. Das Reaktionsgemisch wurde 1,5 Stunden bei Raumtemperatur gerührt. Dann wurden nochmals 2 g Kaliumpermanganat in 50 ml Wasser gelöst, zugetropft und das Gemisch eine weitere Stunde bei Raumtemperatur gerührt. Zur Aufarbeitung wurde dem Reaktionsgemisch festes Natriumbicarbonat portionsweise bis zur Neutralisierung zugesetzt. Dann wurde die Dichlormethanphase abgetrennt und die wäßrige Phase nochmals mit Dichlormethan extrahiert. Die vereinigten Dichlormethanextrakte wurden mit verdünnter Natronlauge und anschließend mit Wasser gewaschen, über Magnesiumsulfat getrocknet und eingedampft. Es wurden 55 g Rohprodukt erhalten. Aus dem Rohprodukt wurde mittels Säulenchromatographie über 1 kg Kieselgel unter leicht erhöhtem Druck (Flash-Chromatographie) unter Verwendung von Cyclohexan/Essigsäureäthylester 1 : 1 als Elutionsmittel das reine 1-Methyl-2-oxo-5-cyclohexyl-2,3-dihydro-1H-1,4-benzodiazepin isoliert, aus Ether kristallisiert und getrocknet. Es wurden 2,3 g Reinprodukt mit einem Schmelzpunkt von 98-100 °C erhalten.

D) 8,9 g des vorstehenden Produktes wurden in 201 ml Toluol suspendiert. Die Suspension wurde auf -20 °C abgekühlt und sodann wurden 9,63 g Kalium-tert.-Butylat unter Rühren zugegeben und das Gemisch noch weitere 15 Minuten gerührt. Dann wurden 5,5 ml Isoamylnitrit so langsam unter Kühlung zugegeben, daß die Temperatur des Reaktionsgemisches unter 0 °C blieb. Anschließend wurde noch 30 Minuten bei 0 °C gerührt. Sodann wurde die Reaktionslösung zur Aufarbeitung unter Rühren in eine Mischung aus 347 ml eiskaltem Wasser, 16,7 ml Eisessig und 347 ml Essigsäureäthylester gegeben. Es wurde kräftig durchgemischt und sodann die organische Phase abgetrennt und die wäßrige Phase nochmals mit Essigsäureäthylester extrahiert. Die vereinigten organischen Phasen wurden mit Wasser gewaschen und eingedampft. Der Rückstand wurde in Toluol aufgenommen und aus Toluol/Äthanol umkristallisiert. Es wurden 12,4 g Rohprodukt erhalten. Dieses wurde durch Flash-Chromatographie über 500 g Kieselgel weiter gereinigt, wobei als Elutionsmittel Cyclohexan/Essigsäureäthylester zunächst im Verhältnis 1 : 1, dann im Verhältnis 4 : 6 verwendet wurde. Nach Kristallisation des erhaltenen Produktes aus Äthanol wurden 4,4 g 3-Hydroxyimino-1-methyl-2-oxo-5-cyclohexyl-2,3-dihydro-1 H-1,4-benzodiazepin mit einem Schmelzpunkt von 205-210 °C erhalten.

E) 4,4 g des vorstehend erhaltenen Produktes wurden in ein Gemisch aus 375 ml Eisessig und 42,3 ml Trifluoressigsäure gegeben. Das Reaktionsgemisch wurde auf 40 °C (Innentemperatur) erwärmt und es wurden insgesamt 2,36 g Zinkstaub portionsweise unter Rühren zugegeben, das Gemisch weitere 2 Stunden bei 40 °C gerührt und sodann nochmals 1,1 g Zinkstaub zugegeben und weitere 1 1/2 Stunden bei 40 °C gerührt. Zur Aufarbeitung wurde das Gemisch mit Toluol verdünnt, abkühlen gelassen und eingedampft. Der verbleibende Rückstand wurde in Dichlormethan aufgenommen, mit wäßriger Natriumcarbonatlösung und Wasser gewaschen, getrocknet und eingedampft. Es wurden 4,0 g rohes 3-Amino-1-methyl-2-oxo-5-cyclohexyl-2,3-dihydro-1H-1,4-benzodiazepin erhalten, welches ohne Reinigung in der nachfolgenden Reaktionsstufe weiterverarbeitet wurde.

F) 2,92 g 4H-Pyrrolo[3,2,1-ij]-5,6-dihydrochinolin-2-carbonsäure (erhalten gemäß Beispiel 1F bis 1G) und 1,99 ml Triäthylamin wurden in 64 ml Dichlormethan gelöst. Die Lösung wurde auf -20 °C abgekühlt und es wurden langsam unter Rühren 1,13 ml Methansulfonsäurechlorid zugetropft und das Reaktionsgemisch 15 Minuten bei -20 °C weitergerührt. Zu der das gemischte Anhydrid aus Methansulfonsäure und der 4H-Pyrrolo[3,2,1-ij]-5,6-dihydro-chinolin-2-carbonsäure enthaltenden Reaktionslösung wurde unter Rühren eine Lösung von 4,0 g des in Stufe E erhaltenen 3-Amino-1-methyl-2-oxo-5-cyclohexyl-2,3-dihydro-1H-1,4-benzodiazepin und 1,99 ml Triäthylamin in 54 ml Dichlormethan bei einer Temperatur zwischen -15 und -20 °C zugetropft, das Reaktionsgemisch weitere 30 Minuten bei -15 °C gerührt und langsam (innerhalb einer Stunde) auf Raumtemperatur erwärmen lassen. Zur Aufarbeitung wurde das Reaktionsgemisch mit Wasser verdünnt, die Dichlormethanphase abgetrennt, mit Natriumbicarbonatlösung und anschließend mit Wasser gewaschen, über Natriumsulfat getrocknet, filtriert und eingedampft. Als Rückstand wurden 7,6 g der rohen Titelverbindung erhalten. Das Rohprodukt wurde mittels Säulenchromatographie an 400 g Kieselgel unter leicht erhöhtem Druck (Flash-Chromatographie) unter Verwendung von Cyclohexan/Essigsäureäthylester 1 : 1 als Elutionsmittel gereinigt. Das gereinigte Produkt wurde aus Äthanol kristallisiert und getrocknet. Es wurden 1,5 g 3-[(4H-Pyrrolo[3,2,1-ij]-5,6-dihydrochinolin-2-carbonyl)-amino]-1-methyl-2-oxo-5-cyclohexyl-1H-2,3-dihydro-1,4-benzodiazepin mit einem Schmelzpunkt von 147-152 °C erhalten.

Beispiel 5:

3-[(8-Fluor-4H-pyrrolo[3,2,1-ij]-5,6-dihydrochinolin-2-carbonyl)-amino]-2-oxo-1-methyl-5-phenyl-1H-2,3-dihydro-1,4-benzodiazepin.

0,99 g 8-Fluor-4H-Pyrrolo[3,2,1-ij]-5,6-dihydrochinolin-2-carbonsäure, 1,4 g 2-Chlor-1-methylpyridiniumjodid, 1,1 ml Triäthylamin und 1,2 g 3-Amino-2-oxo-1-methyl-5-phenyl-2,3-dihydro-1H-1,4-benzodiazepin wurden in 120 ml Dichlormethan gelöst und das Reaktionsgemisch wurde 1 Stunde am Rückfluß gekocht. Zur Aufarbeitung ließ man das Reaktionsgemisch abkühlen, fügte 5 %-ige Natriumbicarbonatlösung hinzu, trennte die organische Phase ab und extrahierte die wäßrige Phase nochmals mit Dichlormethan. Die vereinigten Dichlormethanextrakte wurden über Natriumsulfat getrocknet und das Lösungsmittel wurde unter vermindertem Druck abdestilliert. Die als Rückstand verbleibende rohe Titelverbindung wurde mittels Säulenchromatographie an Kieselgel unter Verwendung von Dichlormethan/Methanol 99 : 1 gereinigt. Es wurden 1,5 g 3-[(8-Fluor-4H-pyrrolo[3,2,1-ij]-5,6-dihydrochinolin-2-carbonyl)-amino]-2-oxo-1-methyl-5-phenyl-1H-2,3-dihydro-1,4-benzodiazepin mit einem Schmelzpunkt von 181-182 °C erhalten.

Beispiel 6:

3-[(4H-Pyrrolo[3,2,1-ij]-5,6-dihydrochinolin-2-carbonyl)-amino]-1-methyl-2-oxo-5-phenyl-1H-2,3-dihydro-1,4-benzodiazepin.

A) 98,6 g 2-Aminobenzophenon wurden in einem Gemisch aus 650 ml Dichlormethan und 50 ml Wasser gelöst. Bei einer Temperatur von -10 °C wurde zu diesem Gemisch eine Lösung von 116,1 g Bromacetylbromid in 150 ml Dichlormethan getropft. Anschließend wurde das Reaktionsgemisch weitere 2 Stunden bei Raumtemperatur gerührt. Zur Aufarbeitung wurde das Reaktionsgemisch mit Wasser versetzt, die organische Phase wurde abgetrennt, nochmals mit Wasser gewaschen, getrocknet und unter vermindertem Druck eingedampft. Das als Rückstand verbleibende Rohprodukt wurde aus Äther/Petroläther kristallisiert. Es wurden 142 g 2-[(2-Bromacetyl)-amino]-benzophenon mit einem Schmelzpunkt von 96-98 °C erhalten.

B) 71 g des vorstehend erhaltenen 2-[(2-Bromacetyl)-amino]-benzophenon wurden in 500 ml Methanol gelöst. Zu dieser Lösung wurde bei einer Temperatur von 10 °C eine Lösung von 75 g Ammoniak in 1,2 l Methanol getropft. Anschließend wurde das Reaktionsgemisch zunächst 1,5 Stunden bei Raumtemperatur gerührt und dann 2 Stunden am Rückfluß erhitzt. Zur Aufarbeitung wurde das Methanol unter vermindertem Druck abdestilliert, der Rückstand in Dichlormethan gelöst, die Lösung mit 10 %-iger wäßriger Natronlauge und mit Wasser gewaschen, getrocknet und unter vermindertem Druck eingeengt. Das als Rückstand verbleibende Rohprodukt wurde aus Methanol kristallisiert. Es wurden 20 g 2-Oxo-5-phenyl-1H-2,3-dihydro-1,4-benzodiazepin mit einem Schmelzpunkt von 178-180 °C erhalten.

C) 60 g des vorstehend erhaltenen Produktes wurden in 1,2 l getrocknetem Tetrahydrofuran gelöst. Unter Feuchtigkeitsausschluß wurden zu der Lösung 34,2 g Kalium-tert.-Butylat zugesetzt. Dann wurde eine Lösung von 20,6 ml Methyljodid in 75 ml Tetrahydrofuran zugetropft und das Reaktionsgemisch noch eine Stunde bei Raumtemperatur gerührt. Zur Aufarbeitung wurde das Reaktionsgemisch mit eiskalter Natriumchloridlösung versetzt, mit Dichlormethan verdünnt, die wäßrige Phase abgetrennt, die organische Phase mit Wasser neutral gewaschen, über Natriumsulfat getrocknet, filtriert und eingedampft. Das als Rückstand erhaltene Rohprodukt wurde aus Äthanol umkristallisiert. Es wurden 56 g 1-Methyl-2-oxo-5-phenyl-1H-2,3-dihydro-1,4-benzodiazepin mit einem Schmelzpunkt von 154-155 °C erhalten.

D) 50,4 g des vorstehend erhaltenen Produktes wurden in 987 ml Toluol gegeben, auf -20 °C abgekühlt und mit 56,4 g Kalium-tert.-Butylat und 32,3 ml Isoamylnitrit nach der in Beispiel 1 D) beschriebenen Methode umgesetzt. Das Reaktionsgemisch wurde wie in Beispiel 1 D) beschrieben aufgearbeitet und das erhaltene Rohprodukt wurde aus Äthanol kristallisiert. Es wurden 47,2 g 3-Hydroxyimino-1-methyl-2-oxo-5-phenyl-2,3-dihydro-1H-1,4-benzodiazepin mit einem Schmelzpunkt von 239-242 °C erhalten.

E) 6,8 g des vorstehend erhaltenen Produktes wurden in 500 ml Methanol gelöst. Zu der Lösung wurden 12 g Raney-Nickel gegeben, und sodann wurde 12 Stunden mit einem Wasserstoffdruck von 6 bar bei Raumtemperatur hydriert. Zur Aufarbeitung wurde vom Katalysator abfiltriert und das Lösungsmittel unter vermindertem Druck abdestilliert. Es wurden 6 g rohes racemisches 3-Amino-1-methyl-2-oxo-5-phenyl-2,3-dihydro-1H-1,4-benzodiazepin erhalten. Dieses wurde zur weiteren Reinigung durch Überführung in sein Benzosulfonatsalz in 50 ml Acetonitril gelöst. Zu der Lösung wurde eine Lösung von 3,6 g Benzolsulfonsäure in 22 ml Acetonitril gegeben und das Reaktionsgemisch eine Stunde bei Raumtemperatur gerührt. Der gebildete kristalline Niederschlag wurde abgesaugt, mit Acetonitril und anschließend mit Hexan gewaschen und dann unter vermindertem Druck getrocknet. Es wurden 5,9 g Benzolsulfonat der 3-Amino-Verbindung mit einem Schmelzpunkt von 224-227 °C erhalten. Zur Freisetzung des Amins wurden 5 g des vorstehend erhaltenen Benzolsulfonat-Salzes in Dichlormethan gelöst und die Lösung mit wäßriger Natriumcarbonatlösung geschüttelt. Die die freigesetzte Aminverbindung enthaltende Dichlormethanphase wurde abgetrennt, getrocknet und eingedampft. Es wurden 3,1 g 3-Amino-1-methyl-2-oxo-5-phenyl-2,3-dihydro-1H-1,4-benzodiazepin erhalten.

F) 0,38 g 4H-Pyrrolo[3,2,1-ij]-5,6-dihydrochinolin-2-carbonsäure, 0,50 g 3-Amino-1-methyl-2-oxo-5-phenyl-2,3-dihydro-1H-1,4-benzodiazepin und 0,46 g Triäthylamin wurden in 50 ml Dichlormethan gelöst. Zu der Lösung wurden 0,58 g 2-Chlor-1-methylpyridiniumjodid gegeben, und das Reaktionsgemisch wurde eine Stunde am Rückfluß gekocht. Anschließend wurde das Reaktionsgemisch wie in Beispiel 5 beschrieben aufgearbeitet. Es wurden 0,69 g 3-[(4H-Pyrrolo[3,2,1-ij]-5,6-dihydrochinolin-2-carbonyl)-amino]-1-methyl-2-oxo-5-phenyl-1H-2,3-dihydro-1,4-benzodiazepin als Schaumharz erhalten.

$^{13}C$-NMR-Spektrum: [Angaben in ppm, (Signalmultiziplität)]

167,88 (s), 167,51 (s), 162,32 (s), 142,91 (s), 138,17 (s), 136,40 (s), 131,93 (d), 130,72 (d), 130,69 (d), 130,23 (s), 129,83 (d), 129,83 (d), 129,22 (s), 128,28 (d), 128,28 (d), 124,58 (d), 124,40 (s), 123,09 (s), 121,60 (d), 120,90 (d), 120,66 (d), 119,34 (d), 104,24 (d), 67,40 (d), 44,38 (t), 35,36 (q), 24,89 (t), 23,13 (t).

Beispiel 7:

Herstellung der optischen Isomeren des 3-[(4H-Pyrrolo[3,2,1-ij]-5,6-dihydrochinolin-2-carbonyl)-amino]-1-methyl-2-oxo-5-phenyl-1H-2,3-dihydro-1,4-benzodiazepins.

7a: (-)-(3S)-3-[(4H-Pyrrolo[3,2,1-ij]-5,6-dihydrochinolin-2-carbonyl)-amino]-1-methyl-2-oxo-5-phenyl-1H-2,3-dihydro-1,4-benzodiazepin.

A) Aus racemischem 3-Amino-1-methyl-2-oxo-5-phenyl-2,3-dihydro-1H-1,4-benzodiazepin (Herstellung siehe Beispiel 6 E) wurde analog Beispiel 2a A-D (-)-(3S)-3-Amino-1-methyl-2-oxo-5-phenyl-1H-2,3-dihydro-1,4-benzodiazepin mit einem optischen Drehwert $[\alpha]_D^{20}$ von -230,8° (c = 1 in Acetolnitril) erhalten.

B) Analog der in Beispiel 1H beschriebenen Methode wurden 1,7 g des vorstehend erhaltenen (-)-(3S)-3-Amino-1-methyl-2-oxo-5-phenyl-2,3-dihydro-1H-1,4-benzodiazepin mit einer das gemischte Anhydrid aus 1,36 g 4H-Pyrrolo[3,2,1-ij]-5,6-dihydrochinolin-2-carbonsäure und 0,52 ml Methansulfonsäurechlorid in Dichlormethan enthaltenden Reaktionslösung umgesetzt. Das Reaktionsgemisch wurde wie in Beispiel 1 H) beschrieben aufgebarbeitet. Es wurden 2,6 g flash-chromatographisch gereinigtes kristallines Produkt erhalten. Dieses wurde zur Entfernung von enatiomeren Verunreinigungen aus Methanol umkristallisiert. Es wurden 1,4 g enantiomerenreines (-)-(3S)-3-[(4H-Pyrrolo[3,2,1-ij]-5,6-dihydrochinolin-2-carbonyl)-amino]-1-methyl-2-oxo-5-phenyl-1H-2,3-dihydro-1,4-benzodiazepin mit einem Schmelzpunkt von 151-158 °C und einem optischen Drehwert $[\alpha]_D^{20}$ von -61,6° (c = 0,5 in Methanol) erhalten.

7b: (+)-(3R)-3-[(4H-Pyrrolo[3,2,1-ij]-5,6-dihydrochinolin-2-carbonyl)-amino]-1-methyl-2-oxo-5-phenyl-1H-2,3-dihydro-1,4-benzodiazepin.

A) Analog Beispiel 2b A-C wurde (+)-(3R)-3-Amino-1-methyl-2-oxo-5-phenyl-2,3-dihydro-1H1,4-benzodiazepin mit einem optischen Drehwert $[\alpha]_D^{20}$ von +259,1 °C (c = 1 in Acetonitril) erhalten.

B) Analog der in Beispiel 1 H) beschriebenen Methode wurden 0,65 g des vorstehend erhaltenen (+)-(3R)-3-Amino-1-methyl-2-oxo-5-phenyl-2,3-dihydro-1H-1,4-benzodiazepin mit einer das gemischte Anhydrid aus 0,52 g 4H-Pyrrolo[3,2,1-ij]-5,6-dihydrochinolin-2-carbonsäure und 0,2 ml Methansulfonsäurechlorid in Dichlormethan enthaltenden Reaktionslösung umgesetzt. Das Reaktionsgemisch wurde wie in Beispiel 1 H) beschrieben aufgearbeitet. Nach Flash-Chromatographie und Kristallisation aus Äthanol wurden 596 mg enantiomerenreines (+)-(3R)-3-[(4H-Pyrrolo[3,2,1-ij]-5,6-dihydrochinolin-2-carbonyl)-amino]-1-methyl-2-oxo-5-phenyl-1H-2,3-dihydro-1,4-benzodiazepin mit einem Schmelzpunkt von 147-156 °C erhalten. Optischer Drehwert $[\alpha]_D^{20}$ = +61,0° (c = 0,5 in Methanol).

Beispiel 8:

3-[(4H-Pyrrolo[3,2,1-ij]-5,6-dihydrochinolin-2-carbonyl)-amino]-1,7-dimethyl-2-oxo-5-(2-thienyl)-1H-2,3-dihydro-1,4-benzodiazepin.

A) 30 g $N_1$-Methyl-$N_1$-(4-methylphenyl)-2-hydroxy-1,3-diaminopropan und 24,2 ml Triäthylamin wurden in 225 ml Dichlormethan gelöst. Unter Eiskühlung wurde in die Lösung langsam eine Lösungvon 16,5 ml Thiophen-2-carbonsäurechlorid in 50 ml Dichlormethan getropft. Das Reaktionsgemisch wurde 12 Stunden bei Raumtemperatur stehengelassen. Zur Aufarbeitung wurde die Reaktionslösung anschließend mit Wasser und mit wäßriger Kochsalzlösung gewaschen, die organische Phase abgetrennt, getrocknet und das Lösungsmittel unter vermindertem Druck abdestilliert. Als Rückstand wurden 52,2 g rohes öliges $N_1$-Methyl-$N_1$-(4-methylphenyl)-$N_2$-(2-thienoyl)-2-hydroxy-1,3-diaminopropan erhalten, welche ohne weitere Reinigung in die nächste Reaktionsstufe eingesetzt wurden.

B) 50,2 g des vorstehend erhaltenen Produktes wurden in 150 ml Phorphoroxychlorid gegeben und das Reaktionsgemisch wurde 90 min. am Rückfluß gekocht. Anschließend wurde abgekühlt und wie in Beispiel 1 B) beschrieben aufgearbeitet. Es wurden 48,5 g eines öligen Rohproduktes erhalten, welches ein Gemisch aus 2-Chlormethyl-1,7-dimethyl-5-(2-thienyl)-2,3-dihydro-1H-1,4-benzodiazepin und 3-Chlor-1,8-dimethyl-6-(2-thienyl)-1,2,3,4-tetrahydro-1,5-benzodiazocin enthielt. Zur Isomerisierung des Benzodiazocin-Anteils wurde das rohe Gemisch in 280 ml Tetrachloräthan gelöst und eine Stunde am Rückfluß gekocht. Anschließend wurde das Tetrachloräthan abgedampft, der Rückstand in Dichlormethan gelöst, die Lösung mit 10 %-iger Natronlauge und anschließend mit Wasser und mit wäßriger Kochsalzlösung gewaschen, getrocknet und unter vermindertem Druck eingedampft. Der verbleibende Rückstand wurde in einem Gemisch aus Methylenchlorid und Methanol gelöst, die Lösung über Magnesiumsilikat (= Florisil[R]) filtriert und eingeengt. Es wurden 32,4 g 2-Chlormethyl-1,7-dimethyl-5-(2-thienyl)2,3-dihydro-1H-1,4-benzodiazepin als ölige Substanz erhalten, welche ohne weitere Reinigung in der nächsten Reaktionsstufe weiterverarbeitet wurde.

C) 13,6 g der vorstehend erhaltenen Substanz wurden nach der in Beispiel 1C beschriebenen Methode mit 8,2 g Kaliumpermanganat oxydiert. Das Reaktionsgemisch wurde wie in Beispiel 1 C) beschrieben aufgearbeitet. Das erhaltene Rohprodukt wurde in Dichlormethan gelöst und mittels Flash-Chromatographie über Kieselgel unter Verwendung von Dichlormethan/Methanol 95 : 5 als Elutionsmittel gereinigt. Es wurden 3,8 g öliges 1,7-Dimethyl-2-oxo-5-(2-thienyl)-2,3-dihydro-1H-1,4 -benzodiazepin erhalten.

D) 7,7 g des vorstehend erhaltenen Produktes wurden in 210 ml Toluol gelöst. Nach Abkühlen auf -20 °C wurden unter $N_2$-Atmosphäre 8,1 g Kalium-tert.-Butylat hinzugefügt und das Gemisch noch weitere 15 min. gerührt. Dann wurden 4,6 ml Isoamylnitrit so langsam unter Kühlung zugegeben, daß die Temperatur des Reaktionsgemisches unter 0 °C blieb. Anschließend wurde noch 30 min. bei 0 °C gerührt. Zur Aufarbeitung wurde das Reaktionsgemisch unter Rühren in eine Mischung von 300 ml eiskaltem Wasser, 300 ml Essigsäureäthylester und 15 ml Eisessig gegeben. Die organische Phase wurde abgetrennt und die wäßrige Phase nochmals mit Essigsäureäthylester extrahiert. Die organischen Phasen wurden vereinigt, mit Wasser und mit wäßriger Kochsalzlösung gewaschen, über Natriumsulfat getrocknet und unter vermindertem Druck eingeengt. Es wurden 6,5 g 3-Hydroxyimino-1,7-dimethyl-2-oxo-5-(2-thienyl)-2,3-dihydro-1H-1,4-benzodiazepin als Schaumharz erhalten.

E) 2,0 g des vorstehend erhaltenen Produktes wurden in 150 ml Methanol gelöst. Es wurden 8 g Raney-Nickel zugegeben, und anschließend wurde 7,5 Stunden mit einem Wasserstoffdruck von 4 bar hydriert.

Zur Aufarbeitung wurde der Katalysator abfiltriert und das Lösungsmittel unter vermindertem Druck abdestilliert. Als Rückstand verblieben 1,4 g Rohprodukt. Dieses wurde in Dichlormethan aufgenommen und mit verdünnter wäßriger Salzsäurelösung extrahiert. Die salzsaure Phase wurde abgetrennt, durch Zugabe von verdünnter wäßriger Natronlauge alkalisch gestellt und mit Dichlormethan extrahiert. Nach Eindampfen des Dichlormethanextraktes wurden 0,7 g 3-Amino-1,7-dimethyl-2-oxo-5-(2-thienyl)-2,3-dihydro-1H-1,4-benzodiazepin als Schauumharz erhalten, welches ohne Reinigung in der nachfolgenden Reaktionsstufe weiterverarbeitet wurde.

F) Analog der in Beispiel 1 H) beschriebenen Methode wurden 1,15 g des vorstehend erhaltenen 3-Amino-1,7-dimethyl-2-oxo-(2-thienyl)-2,3-dihydro-1H-1,4-benzodiazepin mit einer das gemischte Anhydrid aus 0,81 g 4H-Pyrrolo[3,2,1-ij]-5,6-dihydrochinolin-2-carbonsäure und 0,32 ml Methansulfonsäurechlorid in Dichlormethan enthaltenden Reaktionslösung umgesetzt. Das Reaktionsgemisch wurde wie in Beispiel 1 H) beschrieben aufgearbeitet. Es wurden 2,3 g Rohprodukt erhalten, welches in Dichlormethan gelöst und durch Flash-Chromatographie über Kieselgel unter Verwendung von Dichlormethan/Methanol 96 : 4 als Elutionsmittel gereinigt wurde. Nach Abdestillieren des Lösungsmittels wurden 0,7 g Rückstand erhalten, der aus einem Gemisch aus Cyclohexan und Essigsäureäthylester kristallisiert wurde. Es wurden 0,4 g kristallines 3-[(4H-Pyrrolo[3,2,1-ij]-5,6-dihydrochinolin-2-carbonyl)amino]-1,7-dimethyl-2-oxo-5-(2-thienyl)-1H-2,3-dihydro-1,4-benzodiazepin mit einem Schmelzpunkt von 204 - 207 °C erhalten.

Nach den in den vorstehenden Beispielen beschriebenen Verfahren wurden auch die in der folgenden Tabelle aufgeführten Verbindungen der Formel I durch Acylierung der entsprechenden 3-Aminobenzodiazepin-Derivate der Formel II erhalten.

| Bsp. Nr. | $R^1$ | $R^2$ | $R^3$ | $R^4$ = durch $R^7$ und $R^8$ substituiertes Phenyl $R^7$ | $R^8$ | Z | $R^5$ | $R^6$ | Bemerkungen |
|---|---|---|---|---|---|---|---|---|---|
| 9 | $CH_3-$ | 8-$OCH_3$ | H | 2-F | H | $-(CH_2)_3-$ | H | H | Schh. |
| 10 | $CH_3-$ | 7-$CH_3$ | H | 2-F | H | $-(CH_2)_3-$ | H | H | FP: 154-157 |
| 11 | $CH_3-$ | H | H | 2-F | 4-F | $-(CH_2)_3-$ | H | H | FP: 231-234 |
| 12 | $CH_3-$ | H | H | H | H | $-(CH_2)_3-$ | Cl | H | Schh. |
| 13 | $CH_3-$ | 8-$OCH_3$ | H | 2-F | 6-F | $-(CH_2)_3-$ | H | H | FP: 209-210 |
| 14 | $CH_3-$ | 8-$OCH_3$ | H | 2-O-ipen | 4-F | $-(CH_2)_3-$ | H | H | FP: 188-192 |
| 15 | $CH_3-$ | 8-$OCH_3$ | H | 2-F | 4-F | $-(CH_2)_3-$ | H | H | FP: 222-225 |
| 16 | $CH_3-$ | 8-$OCH_3$ | H | 3-Cl | 4-Cl | $-(CH_2)_3-$ | H | H | FP: 207-209 |
| 17 | $CH_3-$ | 8-$OCH_3$ | H | 2-O-ipen | 6-F | $-(CH_2)_3-$ | H | H | FP: 176-178 |
| 18 | $CH_3-$ | 7-$CH_3$ | H | H | H | $-(CH_2)_3-$ | H | H | FP: 231-238 |
| 19 | $CH_3-$ | H | H | 3-Cl | 4-Cl | $-(CH_2)_3-$ | H | H | FP: 273-277 |
| 20 | $CH_3-$ | H | H | 2-F | 6-F | $-(CH_2)_3-$ | H | H | FP: 254-257 |
| 21 | $CH_3-$ | H | H | 2-O-ipen | 6-F | $-(CH_2)_3-$ | H | H | FP: 201-205 |
| 22 | $CH_3-$ | H | H | 2-F | H | $-(CH_2)_3-$ | H | H | FP: 246-251 |
| 23 | Cycpr-$CH_2-$ | H | H | H | H | $-(CH_2)_3-$ | H | H | FP: 145-147 |
| 24 | $CH_3-$ | 7-$CH_3$ | H | 2-O-ipen | H | $-(CH_2)_3-$ | H | H | FP: 189-192 |
| 25 | $(CH_3)_2CH-$ | H | H | H | H | $-(CH_2)_3-$ | H | H | FP: 135-140 |
| 26 | $CH_3-$ | 7-Cl | H | H | H | $-(CH_2)_3-$ | H | 8-F | FP: 263-264 |
| 27 | $CH_3-$ | 7-$CH_3$ | H | 3-Cl | 4-Cl | $-(CH_2)_3-$ | H | H | FP: 173-200 S |
| 28 | $CH_3-$ | 7-$CH_3$ | H | 2-O-ipen | 6-F | $-(CH_2)_3-$ | H | H | FP: 178-181 |
| 29 | $CH_3-$ | 7-$CH_3$ | H | 2-O-ipen | 4-F | $-(CH_2)_3-$ | H | H | FP: 184-188 |
| 30 | $CH_3-$ | H | H | 4-$CH_3$ | H | $-(CH_2)_3-$ | H | H | FP: 148-152 |
| 31 | $CH_3-$ | H | H | H | H | $-(CH_2)_3-$ | H | 8-$OCH_3$ | FP: 252-256 |
| 32 | $CH_3-$ | H | H | H | H | $-(CH_2)_3-$ | H | 9-Cl | FP: 176-179 |
| 33 | $CH_3-$ | H | H | H | H | $-(CH_2)_2-$ | H | H | FP: 193-197 |

| Bsp. Nr. | $R^1$ | $R^2$ | $R^3$ | $R^4$ = durch $R^7$ und $R^8$ substituiertes Phenyl $R^7$ | $R^8$ | Z | $R^5$ | $R^6$ | Bemerkungen |
|---|---|---|---|---|---|---|---|---|---|
| 34 | $CH_3$- | H | H | H | H | $-(CH_2)_2-C(CH_3)_2-$ | H | H | FP: 196-200 |
| 35 | $CH_3$- | H | H | H | H | $-(CH_2)_4-$ | H | H | FP: 236-239 |
| 36 | $CH_3$- | H | H | H | H | -pheneth- | H | H | öl |
| 37 | $CH_3$- | H | H | H | H | $-(CH_2)_2-O-$ | H | H | FP: 213-214 |
| 38 | $CH_3$- | H | H | H | H | $-(CH_2)_2-S-$ | H | H | FP: 255-259 |

ipen = Isoamyl, Cycpr = Cyclopropyl pheneth = phen-1-yl-2-äthylen
Fp. = Schmelzpunkt in °C, Schh. = Schaumharz S = sintern
öl = ölig

Beispiel I:

3-[(4H-Pyrrolo[3,2,1-ij]-5,6-dihydrochinolin-2-carbonyl)-amino]-8-methoxy-1-methyl-2-oxo-5-phenyl-1H-2,3-dihydro-1,4-benzodiazepin enthaltende Tabletten.

Man stellte Tabletten in folgender Zusammensetzung pro Tablette her:

| | |
|---|---|
| 3-[ (4H-Pyrrolo[3,2,1-ij]-5,6-dihydrochinolin-2-carbonyl)-amino]-8-methoxy-1-methyl-2-oxo-5-phenyl-1H-2,3-dihydro-1,4-benzodiazepin | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker wurden mit der 10%-igen Gelatine-Lösung eingedickt. Die Paste wurde zerkleinert und das entstandene Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

Beispiel II:

3-[(4H-Pyrrolo[3,2,1-ij]-5,6-dihydrochinolin-2-carbonyl)-amino]-8-methoxy-1-methyl-2-oxo-5-phenyl-1H-2,3-dihydro-1,4-benzodiazepin enthaltende Tabletten.

Tabletten in folgender Zusammensetzung pro Tablette wurden analog Beispiel I hergestellt.

| | |
|---|---|
| 3-[(4H-Pyrrolo[3,2,1-ij]-5,6-dihydrochinolin-2-carbonyl)-amino]--8-methoxy-1-methyl-2-oxo-5-phenyl-1H-2,3-dihydro-1,4-benzodiazepin | 20 mg |
| Maisstärke | 60 mg |
| Milchzucker | 135 mg |
| Gelatine (als 10%-ige Lösung) | 6 mg |
| Talkum | 5 mg |
| Magnesiumstearat | 5 mg |
| Maisstärke | 9 mg |

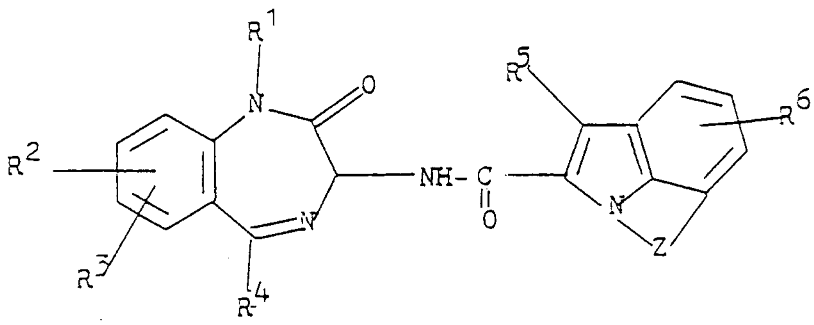

I

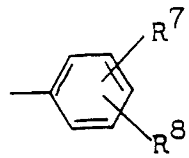

a

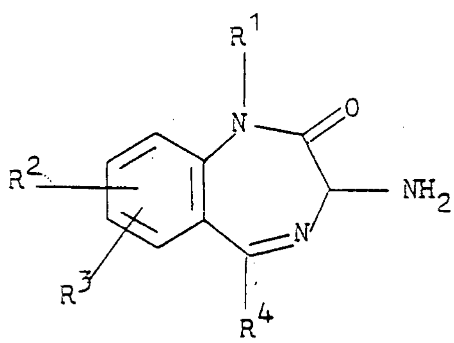

II

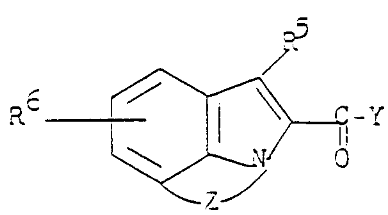

III

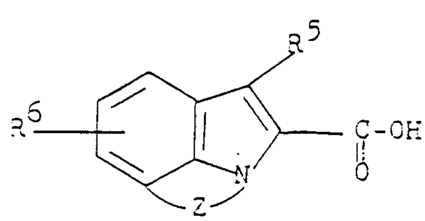

IIIa

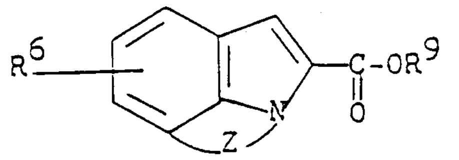

IIIb

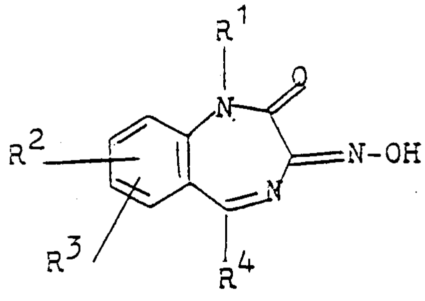

IV

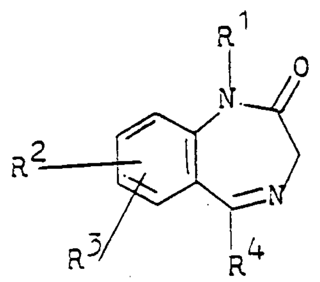

V

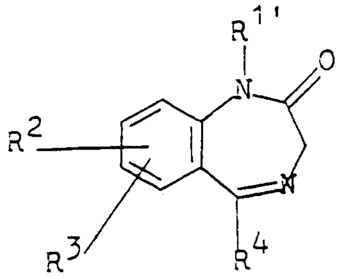

Va

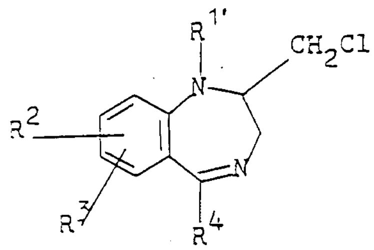

VI

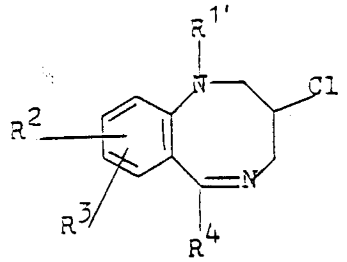

VII

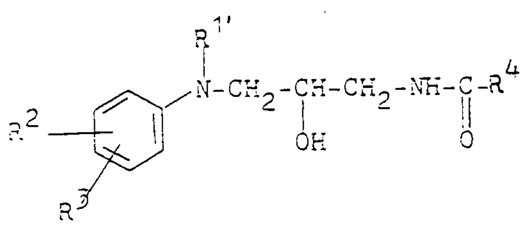

VIII

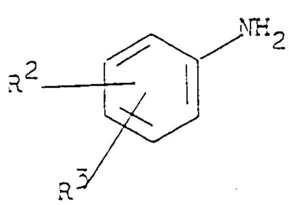

IX

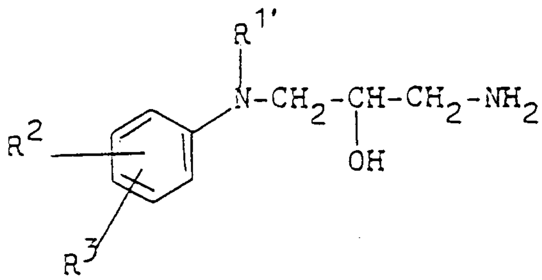

X

$R^4$—CO-Cl

XI

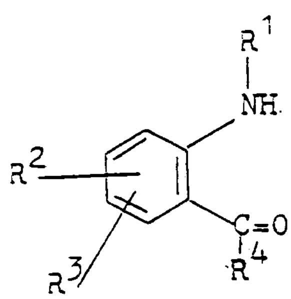

XII

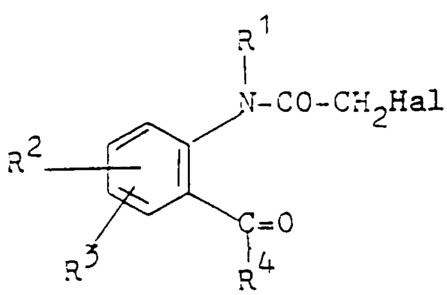

XIII

$R^4$—MgBr XIV

$R^{1'}$-Hal XV

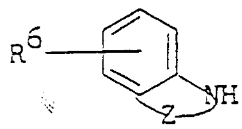

XVI

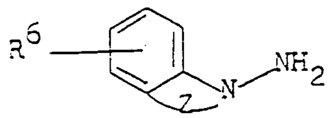

XVII

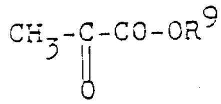

XVIII

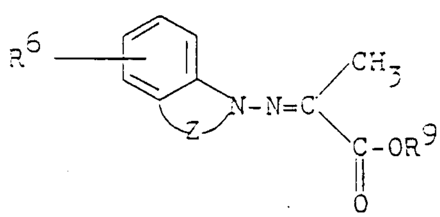

XIX

## Patentansprüche

### Patentansprüche für folgende Vertragsstaaten : AT, BE, CH, DE, DK, FR, GB, IT, LI, LU, NL, SE

**1.** Verbindungen der allgemeinen Formel I

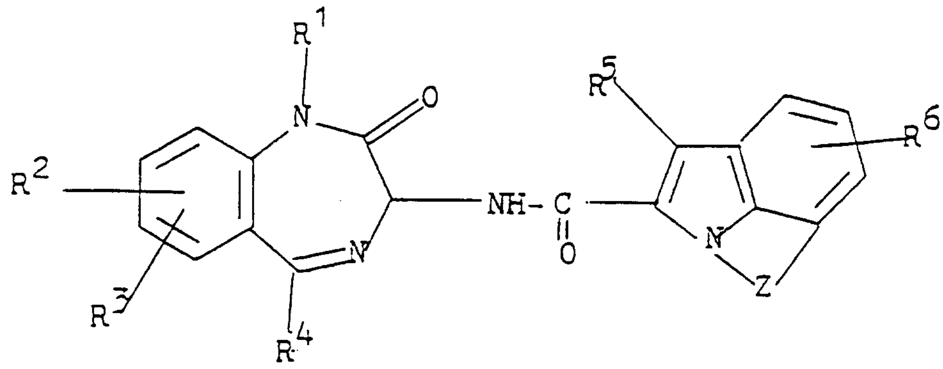

I

worin

$R^1$ Wasserstoff, Alkyl mit 1-4 Kohlenstoffatomen oder Cycloalkylalkyl mit 4-7 Kohlenstoffatomen bedeutet,

$R^2$ Wasserstoff, Halogen, Alkyl mit 1-5 Kohlenstoffatomen, Alkoxy mit 1-5 Kohlenstoffatomen oder Trifluormethyl bedeutet und

$R^3$ Wasserstoff, Halogen, Alkyl mit 1-5 Kohlenstoffatomen oder Alkoxy mit 1-5 Kohlenstoffatomen bedeutet oder

$R^2$ und $R^3$ an zwei benachbarte Kohlenstoffatome gebunden sind und gemeinsam eine Alkylendioxygruppe mit 1-2 Kohlenstoffatomen bedeuten,

$R^4$ für Cycloalkyl mit 5 bis 6 Kohlenstoffatomen, Thiophen oder eine gegebenenfalls substituierte Phenylgruppe a steht,

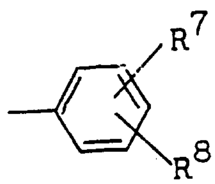

a

worin

$R^7$ Wasserstoff, Halogen, Alkyl mit 1-5 Kohlenstoffatomen, Alkoxy mit 1-5 Kohlenstoffatomen oder Trifluormethyl und

$R^8$ Wasserstoff, Halogen, Alkyl mit 1-5 Kohlenstoffatomen oder Alkoxy mit 1-5 Kohlenstoffatomen bedeuten,

$R^5$ Wasserstoff oder Halogen bedeutet,

$R^6$ Wasserstoff, Halogen, Alkyl mit 1-5 Kohlenstoffatomen, Alkoxy mit 1-5 Kohlenstoffatomen oder Trifluormethyl bedeutet und

Z für eine Alkylenkette mit 2-4 Kohlenstoffatomen, welche gegebenenfalls durch Alkyl mit 1-4 Kohlenstoffatomen mono- oder disubstituiert sein kann oder an welche gegebenenfalls ein 5-6-gliedriger carbocyclischer Ring anelliert sein kann, oder für eine $-X-CH_2-CH_2-$kette, worin X an den Phenylring des Indolgerüstes gebunden ist und Sauerstoff oder Schwefel bedeutet, steht,

sowie deren Säureadditionssalze.

**2.** Verbindungen gemäß Anspruch 1, worin $R^5$ Wasserstoff bedeutet und Z eine Propylenkette darstellt.

**3.** Verbindungen gemäß Anspruch 1, worin $R^4$ eine gegebenenfalls substituierte Phenylgruppe a darstellt, worin $R^7$ und $R^8$ obige Bedeutung besitzen.

**4.** Verbindungen gemäß Anspruch 3, worin Z eine Alkylenkette mit 2-3 Kohlenstoffatomen bedeutet, $R^2$ Wasserstoff, Alkoxy mit 1-5 Kohlenstoffatomen, insbesondere Methoxy, Alkyl mit 1-5 Kohlenstoffatomen, insbesondere Methyl, oder Halogen, insbesondere Chlor, und $R^3$ Wasserstoff bedeuten, $R^7$ Wasserstoff, Alkyl mit 1-5 Kohlenwasserstoffatomen, insbesondere Methyl, oder Halogen, insbesondere Fluor, und $R^8$ Wasserstoff oder Halogen, insbesondere Fluor, bedeuten, $R^5$ Wasserstoff bedeutet und $R^6$ Wasserstoff oder Halogen bedeutet.

**5.** Verbindungen gemäß Anspruch 4, welche 3-[(4H-Pyrrolo(3,2,1-ij]-5,6-dihydrochinolin-2-carbonyl)-amino]-8-methoxy-1-methyl-2-oxo-5-phenyl-1H-2,3-dihydro-1,4-benzodiazepin und dessen Säureadditionssalze und optische Isomere darstellen.

**6.** Verbindungen gemäß Anspruch 2, worin $R^2$ Wasserstoff, Alkoxy mit 1-5 Kohlenstoffatomen, insbesondere Methoxy, Alkyl mit 1-5 Kohlenstoffatomen, insbesondere Methyl, oder Halogen, insbesondere Chlor, und $R^3$ Wasserstoff bedeuten, $R^4$ Cycloalkyl mit 5-6 Kohlenstoffatomen oder Thienyl bedeutet und $R^6$ Wasserstoff oder Halogen bedeutet.

**7.** Verbindungen gemäß Anspruch 6, worin $R^4$ Cyclohexyl bedeutet.

**8.** Arzneimittel, enthaltend eine pharmakologisch wirksame Menge einer Verbindung gemäß Anspruch 1 und übliche pharmazeutische Hilfs- und Trägerstoffe.

**9.** Verfahren zur Herstellung von Verbindungen der allgemeinen Formel I gemäß Anspruch 1

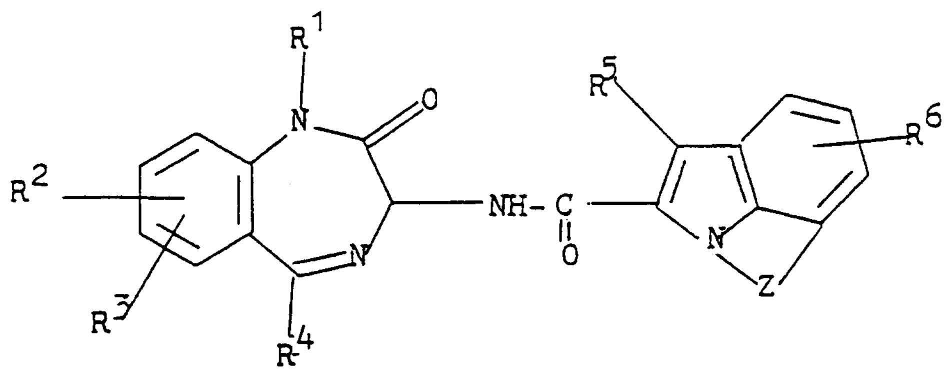

I

worin $R^1$, $R^2$, $R^3$, $R^4$, $R^5$, $R^6$ und Z die in Anspruch 1 angegebene Bedeutung besitzen sowie deren Säureadditionssalzen, dadurch gekennzeichnet, daß man Amino-Verbindungen der allgemeinen Formel II

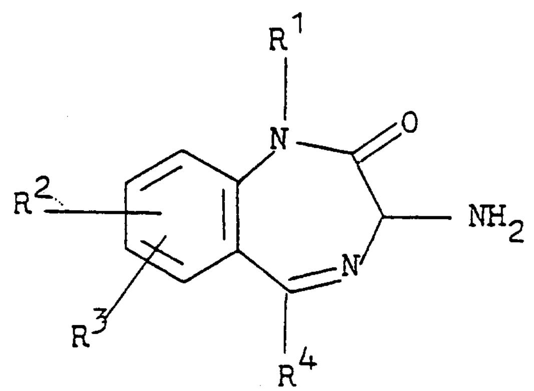

II

worin $R^1$, $R^2$, $R^3$ und $R^4$ obige Bedeutung besitzen, mit Säuren oder reaktionsfähigen Säurederivaten der allgemeinen Formel III

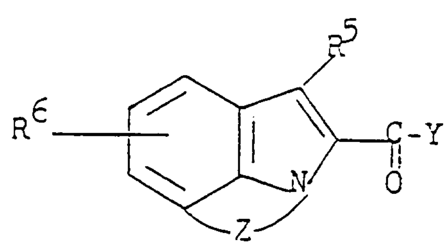

III

worin $R^5$, $R^6$ und Z obige Bedeutung besitzen und Y Hydroxy oder eine reaktive Gruppe bedeutet, acyliert und gegebenenfalls freie Verbindungen der Formel I in ihre Säureadditionssalze überführt oder die Säureadditionssalze in die freien Verbindungen der Formel I überführt.

**Patentansprüche für folgende Vertragsstaaten : ES, GR**

1. Verfahren zur Herstellung von Verbindungen der allgemeinen Formel I

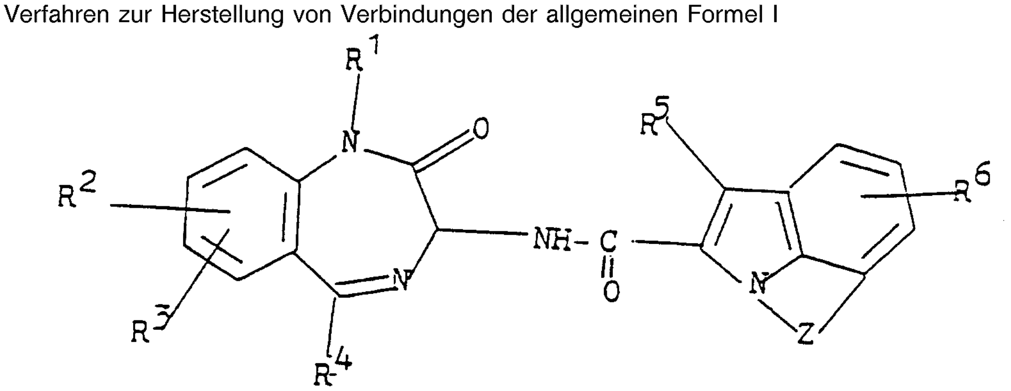

I

worin

| | |
|---|---|
| $R^1$ | Wasserstoff, Alkyl mit 1-4 Kohlenstoffatomen oder Cycloalkylalkyl mit 4-7 Kohlenstoffatomen bedeutet, |
| $R^2$ | Wasserstoff, Halogen, Alkyl mit 1-5 Kohlenstoffatomen, Alkoxy mit 1-5 Kohlenstoffatomen oder Trifluormethyl bedeutet und |
| $R^3$ | Wasserstoff, Halogen, Alkyl mit 1-5 Kohlenstoffatomen oder Alkoxy mit 1-5 Kohlenstoffatomen bedeutet oder |
| $R^2$ und $R^3$ | an zwei benachbarte Kohlenstoffatome gebunden sind und gemeinsam eine Alkylendioxygruppe mit 1-2 Kohlenstoffatomen bedeuten, |
| $R^4$ | für Cycloalkyl mit 5 bis 6 Kohlenstoffatomen, Thiophen oder eine gegebenenfalls substituierte Phenylgruppe a steht, |

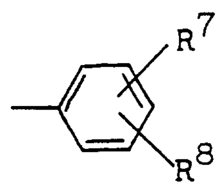

a

worin

| | |
|---|---|
| $R^7$ | Wasserstoff, Halogen, Alkyl mit 1-5 Kohlenstoffatomen, Alkoxy mit 1-5 Kohlenstoffatomen oder Trifluormethyl und |
| $R^8$ | Wasserstoff, Halogen, Alkyl mit 1-5 Kohlenstoffatomen oder Alkoxy mit 1-5 Kohlenstoffatomen bedeuten, |
| $R^5$ | Wasserstoff oder Halogen bedeutet, |
| $R^6$ | Wasserstoff, Halogen, Alkyl mit 1-5 Kohlenstoffatomen, Alkoxy mit 1-5 Kohlenstoffatomen oder Trifluormethyl bedeutet und |
| Z | für eine Alkylenkette mit 2-4 Kohlenstoffatomen, welche gegebenenfalls durch Alkyl mit 1-4 Kohlenstoffatomen mono- oder disubstituiert sein kann oder an welche gegebenenfalls ein 5-6-gliedriger carbocyclischer Ring anelliert sein kann, oder für eine $-X-CH_2-CH_2-$kette, worin X an den Phenylring des Indolgerüstes gebunden ist und Sauerstoff oder Schwefel bedeutet, steht, |

sowie deren Säureadditionssalzen, dadurch gekennzeichnet, daß man Amino-Verbindungen der allgemeinen Formel II

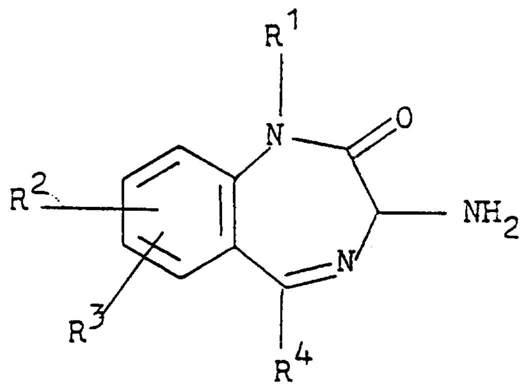

II

worin $R^1$, $R^2$, $R^3$ und $R^4$ obige Bedeutung besitzen, mit Säuren oder reaktionsfähigen Säurederivaten der allgemeinen Formel III

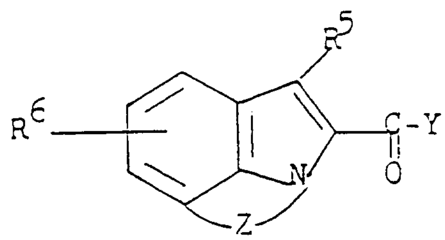

III

worin $R^5$, $R^6$ und Z obige Bedeutung besitzen und Y Hydroxy oder eine reaktive Gruppe bedeutet, acyliert und gegebenenfalls freie Verbindungen der Formel I in ihre Säureadditionssalze überführt oder die Säureadditionssalze in die freien Verbindungen der Formel I überführt.

**2.** Verfahren gemäß Anspruch 1, dadurch gekennzeichnet, daß Verbindungen, worin $R^5$ Wasserstoff bedeutet und Z eine Propylenkette darstellt, hergestellt werden.

**3.** Verfahren gemäß Anspruch 1, dadurch gekennzeichnet, daß Verbindungen, worin $R^4$ eine gegebenenfalls substituierte Phenylgruppe a darstellt, worin $R^7$ und $R^8$ obige Bedeutung besitzen, hergestellt werden.

**4.** Verfahren gemäß Anspruch 3, dadurch gekennzeichnet, daß Verbindungen hergestellt werden, worin Z eine Alkylenkette mit 2-4 Kohlenstoffatomen bedeutet, $R^2$ Wasserstoff, Alkoxy mit 1-5 Kohlenstoffatomen, insbesondere Methoxy, Alkyl mit 1-5 Kohlenstoffatomen, insbesondere Methyl, oder Halogen, insbesondere Chlor, und $R^3$ Wasserstoff bedeuten, $R^7$ Wasserstoff, Alkyl mit 1-5 Kohlenstoffatomen, insbesondere Methyl, oder Halogen, insbesondere Fluor, und $R^8$ Wasserstoff oder Halogen, insbesondere Fluor, bedeuten, $R^5$ Wasserstoff bedeutet und $R^6$ Wasserstoff oder Halogen bedeutet.

**5.** Verfahren gemäß Anspruch 4, dadurch gekennzeichnet, daß 3-[(4H-Pyrrolo[3,2,1-ij]-5,6-dihydrochinolin-2-carbonyl)-amino]-8-methoxy-1-methyl-2-oxo-5-phenyl-1H-2,3-dihydro-1,4-benzodiazepin und dessen Säureadditionssalze und optische Isomere hergestellt werden.

**6.** Verfahren gemäß Anspruch 2, dadurch gekennzeichnet, daß Verbindungen hergestellt werden, worin $R^2$ Wasserstoff, Alkoxy mit 1-5 Kohlenstoffatomen, insbesondere Methoxy, Alkyl mit 1-5 Kohlenstoffatomen, insbesondere Methyl, oder Halogen, insbesondere Chlor, und $R^3$ Wasserstoff bedeuten, $R^4$ Cycloalkyl mit 5-6 Kohlenstoffatomen oder Thienyl bedeutet und $R^6$ Wasserstoff oder Halogen bedeutet.

**7.** Verfahren gemäß Anspruch 6, dadurch gekennzeichnet, daß Verbindungen, worin $R^4$ Cyclohexyl bedeutet, hergestellt werden.

## Claims

**Claims for the following Contracting States : AT, BE, CH, DE, DK, FR, GB, IT, LI, LU, NL, SE**

1. Compounds of the general formula I

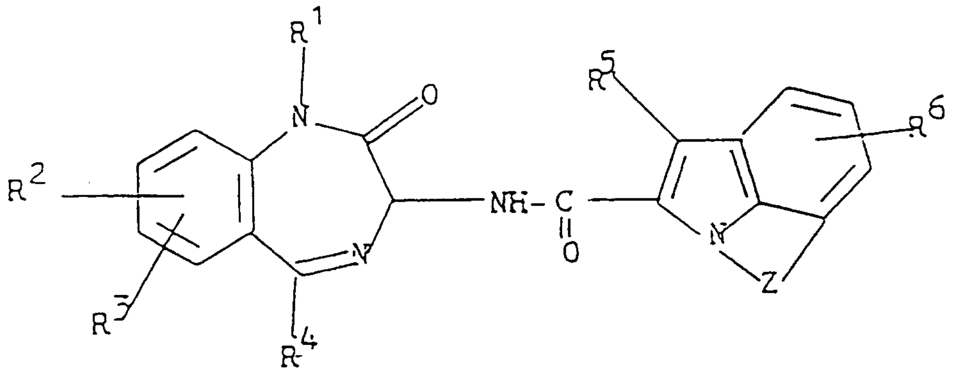

I

in which

| | |
|---|---|
| $R^1$ | denotes hydrogen, alkyl with 1-4 carbon atoms or cycloalkylalkyl with 4-7 carbon atoms, |
| $R^2$ | denotes hydrogen, halogen, alkyl with 1-5 carbon atoms, alkoxy with 1-5 carbon atoms or trifluoromethyl, and |
| $R^3$ | denotes hydrogen, halogen, alkyl with 1-5 carbon atoms or alkoxy with 1-5 carbon atoms, or |
| $R^2$ and $R^3$ | are bonded to two adjacent carbon atoms and together denote an alkylenedioxy group with 1-2 carbon atoms, |
| $R^4$ | stands for cycloalkyl with 5 to 6 carbon atoms, thiophene or an optionally substituted phenyl group a |

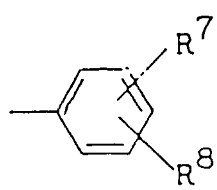

a

in which

| | |
|---|---|
| $R^7$ | denotes hydrogen, halogen, alkyl with 1-5 carbon atoms, alkoxy with 1-5 carbon atoms or trifluoromethyl, and |
| $R^8$ | denotes hydrogen, halogen, alkyl with 1-5 carbon atoms or alkoxy with 1-5 carbon atoms, |
| $R^5$ | denotes hydrogen or halogen, |
| $R^6$ | denotes hydrogen, halogen, alkyl with 1-5 carbon atoms, alkoxy with 1-5 carbon atoms or trifluoromethyl, and |
| Z | stands for an alkylene chain with 2-4 carbon atoms, which can optionally be mono- or disubstituted by alkyl with 1-4 carbon atoms, or onto which a 5-6-member carbocyclic ring can optionally be fused, or for an $-X-CH_2-CH_2-$ chain in which X is bonded to the phenyl ring of the indole framework and denotes oxygen or sulphur, |

and the acid addition salts thereof.

2. Compounds according to Claim 1, in which $R^5$ denotes hydrogen and Z represents a propylene chain.

3. Compounds according to Claim 1, in which $R^4$ represents an optionally substituted phenyl group a in which $R^7$ and $R^8$ have the above meanings.

4. Compounds according to Claim 3, in which Z denotes an alkylene chain with 2-3 carbon atoms, $R^2$ denotes hydrogen, alkoxy with 1-5 carbon atoms, in particular methoxy, alkyl with 1-5 carbon atoms, in particular methyl, or halogen, in particular chlorine, and $R^3$ denotes hydrogen, $R^7$ denotes hydrogen, alkyl with 1-5 carbon atoms, in particular methyl, or halogen, in particular fluorine, and $R^8$ denotes hydrogen or halogen, in particular fluorine, $R^5$ denotes hydrogen, and $R^6$ denotes hydrogen or halogen.

5. Compounds according to Claim 4 which represent 3-[(4H-pyrrolo[3,2,1-ij]-5,6-dihydroquinoline-2-carbonyl)-amino]-8-methoxy-1-methyl-2-oxo-5-phenyl-1H-2,3-dihydro-1,4-benzodiazepine and the acid addition salts and optical isomers thereof.

6. Compounds according to Claim 2, in which $R^2$ denotes hydrogen, alkoxy with 1-5 carbon atoms, in particular methoxy, alkyl with 1-5 carbon atoms, in particular methyl, or halogen, in particular chlorine, and $R^3$ denotes hydrogen, $R^4$ denotes cycloalkyl with 5-6 carbon atoms or thienyl, and $R^6$ denotes hydrogen or halogen.

7. Compounds according to Claim 6, in which $R^4$ denotes cyclohexyl.

8. Medicaments, containing a pharmacologically effective amount of a compound according to Claim 1 and customary pharmaceutical auxiliaries and vehicles.

9. A process for the preparation of compounds of the general formula I in accordance with Claim 1

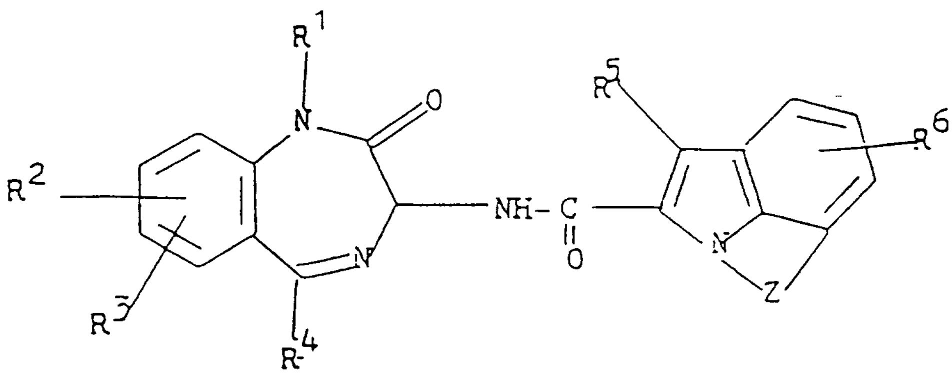

I

in which
$R^1$, $R^2$, $R^3$, $R^4$, $R^5$, $R^6$ and Z have the meanings given in Claim 1, and the acid addition salts thereof, characterised in that amino compounds of the general formula II

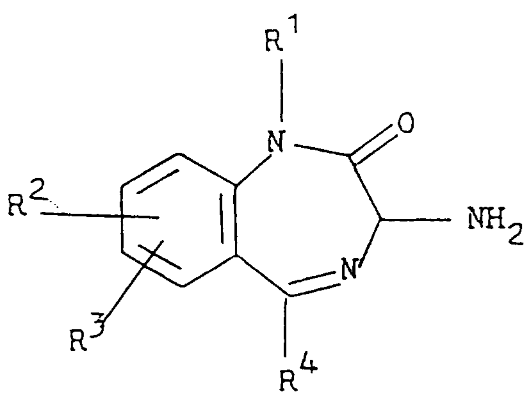

II

in which $R^1$, $R^2$, $R^3$ and $R^4$ have the above meanings, are acylated with acids or reactive acid derivatives of the general formula III

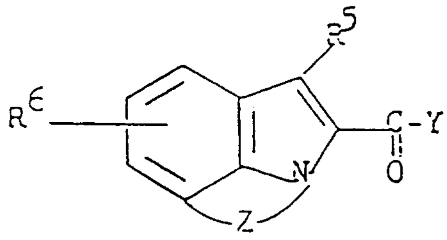

III

in which $R^5$, $R^6$ and Z have the above meanings and Y denotes hydroxy or a reactive group, and, optionally, free compounds of Formula I are converted into the acid addition salts thereof, or the acid addition salts are converted into the free compounds of Formula I.

**Claims for the following Contracting States : ES, GR**

**1.** A process for the preparation of compounds of the general formula I

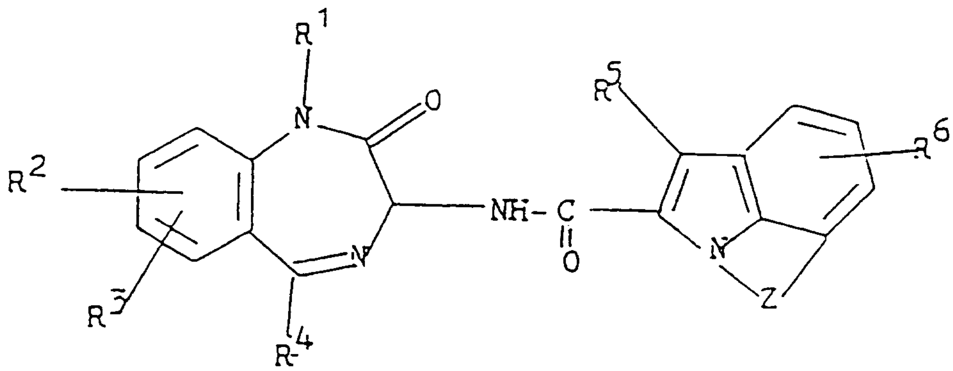

I

in which

| | |
|---|---|
| $R^1$ | denotes hydrogen, alkyl with 1-4 carbon atoms or cycloalkylalkyl with 4-7 carbon atoms, |
| $R^2$ | denotes hydrogen, halogen, alkyl with 1-5 carbon atoms, alkoxy with 1-5 carbon atoms or trifluoromethyl, and |
| $R^3$ | denotes hydrogen, halogen, alkyl with 1-5 carbon atoms or alkoxy with 1-5 carbon atoms, or |
| $R^2$ and $R^3$ | are bonded to two adjacent carbon atoms and together denote an alkylenedioxy group with 1-2 carbon atoms, |
| $R^4$ | stands for cycloalkyl with 5 to 6 carbon atoms, thiophene or an optionally substituted phenyl group a |

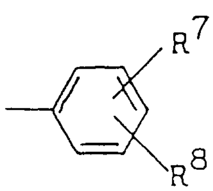

a

in which

| | |
|---|---|
| $R^7$ | denotes hydrogen, halogen, alkyl with 1-5 carbon atoms, alkoxy with 1-5 carbon atoms or trifluoromethyl, and |
| $R^8$ | denotes hydrogen, halogen, alkyl with 1-5 carbon atoms or alkoxy with 1-5 carbon atoms, |
| $R^5$ | denotes hydrogen or halogen, |
| $R^6$ | denotes hydrogen, halogen, alkyl with 1-5 carbon atoms, alkoxy with 1-5 carbon atoms or trifluoromethyl, and |

Z stands for an alkylene chain with 2-4 carbon atoms, which can optionally be mono- or disubstituted by alkyl with 1-4 carbon atoms, or onto which a 5-6-member carbocyclic ring can optionally be fused, or for an $-X-CH_2-CH_2-$ chain in which X is bonded to the phenyl ring of the indole framework and denotes oxygen or sulphur,

and the acid addition salts thereof, characterised in that amino compounds of the general formula II

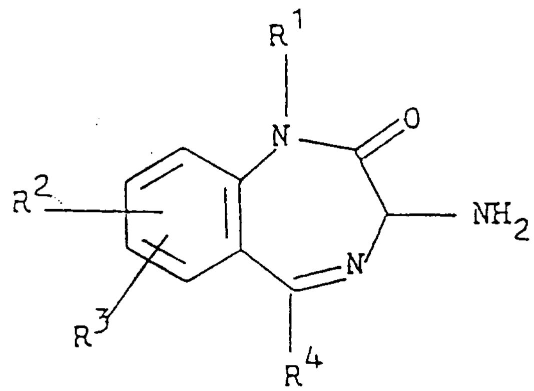

II

in which $R^1$, $R^2$, $R^3$ and $R^4$ have the above meanings, are acylated with acids or reactive acid derivatives of the general formula III

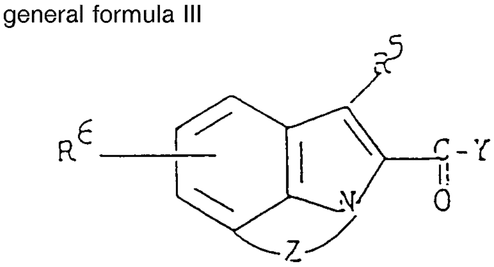

III

in which $R^5$, $R^6$ and Z have the above meanings and Y denotes hydroxy or a reactive group, and, optionally, free compounds of Formula I are converted into the acid addition salts thereof, or the acid addition salts are converted into the free compounds of Formula I.

**2.** A process according to Claim 1, characterised in that compounds in which $R^5$ denotes hydrogen, and Z represents a propylene chain, are produced.

**3.** A process according to Claim 1, characterised in that compounds in which $R^4$ represents an optionally substituted phenyl group a in which $R^7$ and $R^8$ have the above meanings are produced.

**4.** A process according to Claim 3, characterised in that compounds are produced in which Z denotes an alkylene chain with 2-4 carbon atoms, $R^2$ denotes hydrogen, alkoxy with 1-5 carbon atoms, in particular methoxy, alkyl with 1-5 carbon atoms, in particular methyl, or halogen, in particular chlorine, and $R^3$ denotes hydrogen, $R^7$ denotes hydrogen, alkyl with 1-5 carbon atoms, in particular methyl, or halogen, in particular fluorine, and $R^8$ denotes hydrogen or halogen, in particular fluorine, $R^5$ denotes hydrogen, and $R^6$ denotes hydrogen or halogen.

**5.** A process according to Claim 4, characterised in that 3-[(4H-pyrrolo[3,2,1-ij]-5,6-dihydroquinoline-2-carbonyl)-amino]-8-methoxy-1-methyl-2-oxo-5-phenyl-1H-2,3-dihydro-1,4-benzodiazepine and the acid addition salts and optical isomers thereof are produced.

**6.** A process according to Claim 2, characterised in that compounds are produced in which $R^2$ denotes hydrogen, alkoxy with 1-5 carbon atoms, in particular methoxy, alkyl with 1-5 carbon atoms, in particular methyl, or halogen, in particular chlorine, and $R^3$ denotes hydrogen, $R^4$ denotes cycloalkyl with 5-6 carbon atoms or thienyl, and $R^6$ denotes hydrogen or halogen.

7. A process according to Claim 6, characterised in that compounds in which $R^4$ denotes cyclohexyl are produced.

**Revendications**

**Revendications pour les Etats contractants suivants : AT, BE, CH, DE, DK, FR, GB, IT, LI, LU, NL, SE**

1. Composés de formule générale I

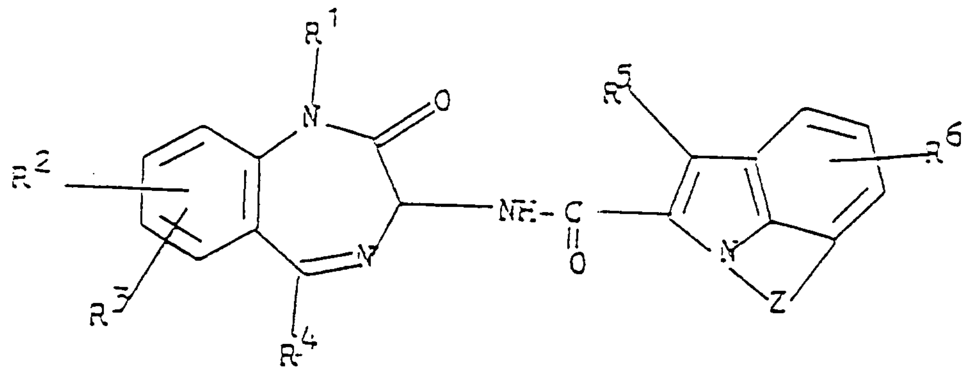

I

dans laquelle :

$R^1$ représente un atome d'hydrogène, un groupe alkyle ayant 1 à 4 atomes de carbone ou cycloalkylalkyle ayant 4 à 7 atomes de carbone,

$R^2$ représente un atome d'hydrogène ou d'halogène, un groupe alkyle ayant 1 à 5 atomes de carbone, alcoxy ayant 1 à 5 atomes de carbone ou trifluorométhyle,et

$R^3$ représente un atome d'hydrogène ou d'halogène, un groupe alkyle ayant 1 à 5 atomes de carbone ou alcoxy ayant 1 à 5 atomes de carbone, ou bien

$R^2$ et $R^3$ sont fixés sur deux atomes de carbone voisins et forment ensemble un groupe alkylènedioxy comportant 1 ou 2 atomes de carbone,

$R^4$ représente un groupe cycloalkyle ayant 5 à 6 atomes de carbone, un groupe thiophène ou un groupe phényle a éventuellement substitué,

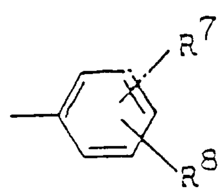

a

dans lequel,

$R^7$ représente un atome d'hydrogène ou d'halogène, un groupe alkyle ayant 1 à 5 atomes de carbone, alcoxy ayant 1 à 5 atomes de carbone ou trifluorométhyle, et

$R^8$ représente un atome d'hydrogène ou d'halogène, un groupe alkyle ayant 1 à 5 atomes de carbone ou alcoxy ayant 1 à 5 atomes de carbone,

$R^5$ représente un atome d'hydrogène ou d'halogène,

$R^6$ représente un atome d'hydrogène ou d'halogène ou un groupe alkyle ayant 1 à 5 atomes de carbone, alcoxy ayant 1 à 5 atomes de carbone ou trifluorométhyle, et

Z représente une chaîne alkylène ayant 2 à 4 atomes de carbone, qui peut éventuellement être monosubstituée ou disubstituée par un groupe alkyle ayant 1 à 4 atomes de carbone, ou sur laquelle peut être éventuellement condensé un noyau carbocyclique pentagonal ou hexagonal, ou bien Z représente une chaîne $-X-CH_2-CH_2-$, dans laquelle X est fixé sur le noyau phényle du squelette indole et représente un atome d'oxygène ou de soufre, ainsi que leurs sels d'addition d'acide.

2. Composés selon la revendication 1, dans lesquels $R^5$ représente un atome d'hydrogène.et Z représente une chaîne propylène.

3. Composés selon la revendication 1, dans lesquels $R^4$ représente un groupe phényle a éventuellement substitué, dans lequel $R^7$ et $R^8$ ont le sens précité.

4. Composés selon la revendication 3, dans lesquels Z représente une chaîne alkylène ayant 2 ou 3 atomes de carbone; $R^2$ représnete un atome d'hydrogène, un groupe alcoxy ayant 1 à 5 atomes de carbone, notamment un groupe méthoxy, alkyle ayant 1 à 5 atomes de carbone, notamment méthyle ou, un atome d'halogène, notamment le chlore; et $R^3$ représente un atome d'hydrogène; $R^7$ représente un atome d'hydrogène, un groupe alkyle ayant 1 à 5 atomes de carbone, notamment un groupe méthyle, ou un atome d'halogène, notamment le fluor; et $R^8$ représente un atome d'hydrogène ou d'halogène, notamment un atome de fluor; $R^5$ représente un atome d'hydrogène et $R^6$ représente un atome d'hydrogène ou d'halogène.

5. Composés selon la revendication 4, qui sont la 3-[(4H-pyrrolo(3,2,1-ij)-5,6-dihydroquinoléine-2-carbonyl)-amino]-8-méthoxy-1-méthyl-2-oxo-5-phényl-1H-2,3-di- hydro-1,4-benzodiazépine et ses sels d'addition d'acide et isomères optiques.

6. Composés selon la revendication 2, dans lesquels $R^2$ représente un atome d'hydrogène, un groupe alcoxy ayant 1 à 5 atomes de carbone, notamment méthoxy, alkyle ayant 1 à 5 atomes de carbone, notamment méthyle, ou un atome d'halogène, notamment le chlore et $R^3$ représente un atome d'hydrogène; $R^4$ représente un groupe cycloalkyle ayant 5 à 6 atomes de carbone ou un groupe thiényle, et $R^6$ représente un atome d'hydrogène ou d'halogène.

7. Composés selon la revendication 6, dans lesquels $R^4$ représente un groupe cyclohexyle.

8. Médicament, contenant une quantité pharmacologiquement active d'un composé selon la revendication 1 et des adjuvants et excipients ou véhicules de support pharmaceutiques usuels.

9. Procédé de préparation de composés de formule générale I selon la revendication I :

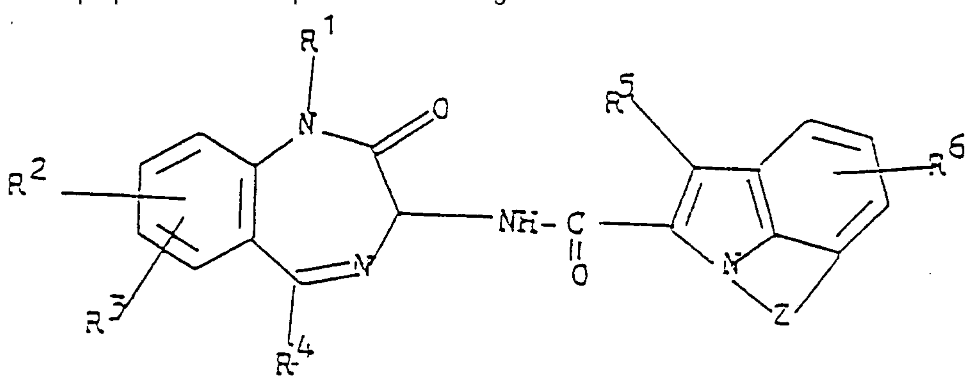

I

dans laquelle $R^1$, $R^2$, $R^3$, $R^4$, $R^5$, $R^6$ et Z ont le sens indiqué à la revendication 1, ainsi que de leurs sels d'addition d'acide, procédé caractérisé en ce qu'on acyle des amines de formule générale II

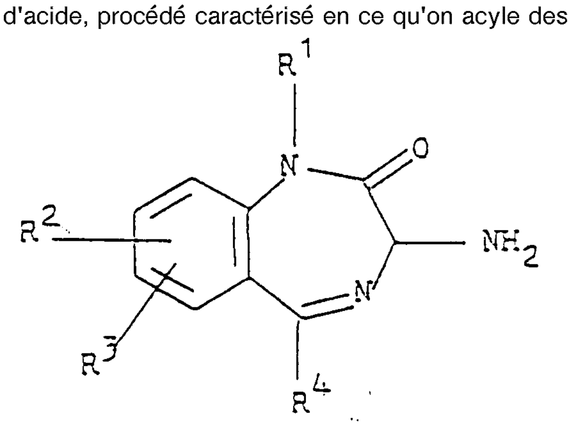

II

(dans laquelle $R^1$, $R^2$, $R^3$ et $R^4$ ont le sens ci-dessus) avec des acides ou des dérivés réactifs d'acides de formule générale III :

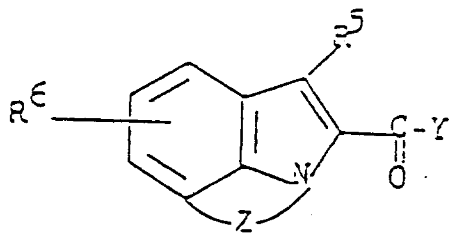

III

(dans laquelle $R^5$, $R^6$ et Z ont le sens ci-dessus, et Y représente un groupe hydroxyle ou un groupe réactif) et l'on transforme éventuellement les composés libres de formule 1 en leurs sels d'addition d'acide ou bien on transforme les sels d'addition d'acide en les composés libres de formule I.

**Revendications pour les Etats contractants suivants : ES, GR**

**1.** Procédé de préparation de composés de formule générale I :

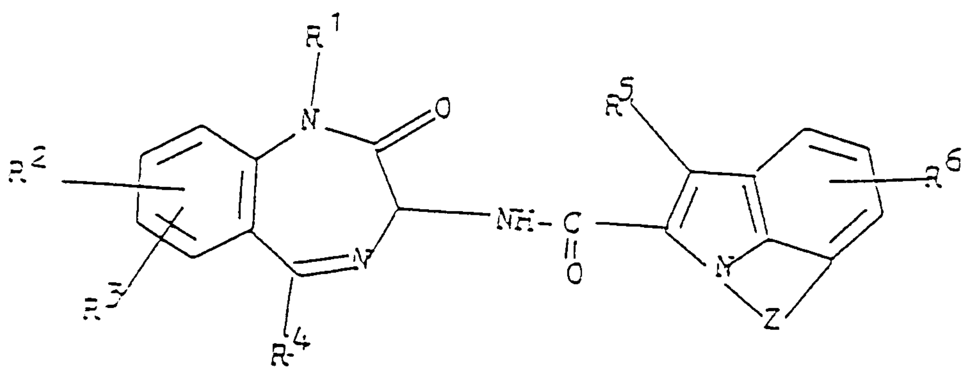

I

dans laquelle :

$R^1$ représente un atome d'hydrogène, un groupe alkyle ayant 1 à 4 atomes de carbone ou cycloalkylalkyle ayant 4 à 7 atomes de carbone,

$R^2$ représente un atome d'hydrogène ou d'halogène, un groupe alkyle ayant 1 à 5 atomes de carbone, alcoxy ayant 1 à 5 atomes de carbone ou trifluorométhyle, et

$R^3$ représente un atome d'hydrogène ou d'halogène, un groupe alkyle ayant 1 à 5 atomes de carbone ou alcoxy ayant 1 à 5 atomes de carbone, ou bien

$R^2$ et $R^3$ sont fixés sur deux atomes de carbone voisins et forment ensemble un groupe alkylènedioxy comportant 1 ou 2 atomes de carbone,

$R^4$ représente un groupe cycloalkyle ayant 5 à 6 atomes de carbone, un groupe thiophène ou un groupe phényle a éventuellement substitué.

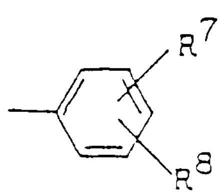

a

dans lequel: $R^7$ représente un atome d'hydrogène ou d'halogène, un groupe alkyle ayant 1 à 5 atomes de carbone, alcoxy ayant 1 à 5 atomes de carbone ou trifluorométhyle, et

$R^8$ représente un atome d'hydrogène ou d'halogène, un groupe alkyle ayant 1 à 5 atomes de carbone ou alcoxy ayant 1 à 5 atomes de carbone; $R^5$ représente un atome d'hydrogène ou d'halogène,

$R^6$ représente un atome d'hydrogène ou d'halogène ou un groupe alkyle ayant 1 à 5 atomes de carbone, alcoxy ayant 1 à 5 atomes de carbone ou trifluorométhyle, et

Z représente une chaîne alkylène ayant 2 à 4 atomes de carbone, qui peut éventuellement être monosubstituée ou disubstituée par un groupe alkyle ayant 1 à 4 atomes de carbone, ou sur laquelle peut être éventuellement condensé un noyau carbocyclique pentagonal ou hexagonal, ou bien Z représente une chaîne $-X-CH_2-CH_2-$, dans laquelle X est fixé sur le noyau phényle du squelette indole et représente un atome d'oxygène ou de soufre, ainsi que leurs sels d'addition d'acide, procédé caractérisé en ce qu'on acyle des amines de formule générale II

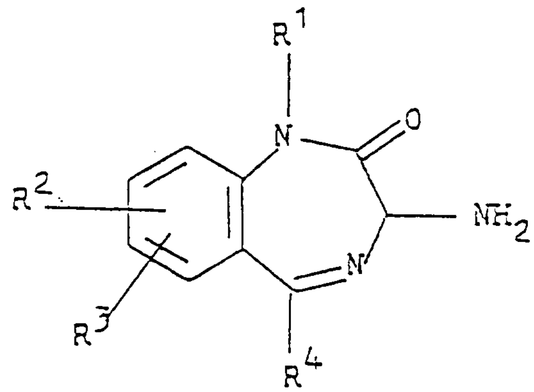

II

(dans laquelle $R^1$, $R^2$, $R^3$ et $R^4$ ont le sens ci-dessus) avec des acides ou des dérivés réactifs d'acide de formule générale III :

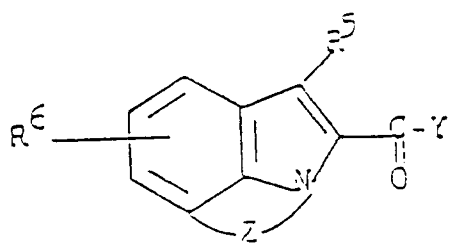

III

(dans laquelle $R^5$, $R^6$ et Z ont le sens ci-dessus, et Y représente un groupe hydroxyle ou un groupe réactif) et l'on transforme éventuellement les composés libres de formule 1 en leurs sels d'addition d'acide ou bien on transforme les sels d'addition d'acide en les composés libres de formule I.

**2.** Procédé selon la revendication 1, caractérisé en ce qu'on prépare des composés dans lesquels $R^5$ représente un atome d'hydrogène et Z représente une chaîne propylène.

**3.** Procédé selon la revendication 1, caractérisé en ce qu'on prépare des composés dans lesquels $R^4$ représente un groupe phényle a éventuellement substitué, et dans lequel $R^7$ et $R^8$ ont le sens précité.

**4.** Procédé selon la revendication 3, caractérisé en ce qu'on prépare des composés dans lesquels Z représente une chaîne alkylène ayant 2 à 4 atomes de carbone; $R^2$ représente un atome d'hydrogène, un groupe alcoxy ayant 1 à 5 atomes de carbone, notamment méthoxy, un groupe alkyle ayant 1 à 5 atomes de carbone, en particulier méthyle, ou un halogène, en particulier du chlore, et $R^3$ représente un atome d'hydrogène, $R^7$ représene un atome d'hydrogène, un groupe alkyle ayant 1 à 5 atomes de carbone, notamment un groupe méthyle, ou un atome d'halogène, en particulier le fluor, et $R^8$ représente un atome d'hydrogène ou d'halogène, notamment de fluor; $R^5$ représente un atome d'hydrogène et $R^6$ représente un atome d'hydrogène ou d'halogène.

**5.** Procédé selon la revendication 4, caractérisé en ce qu'on prépare la 3-[(4H-pyrrolo[3,2,1-ij]-5,6-dihydroquinoléine-2-carbonyl)-amino]-8-méthoxy-1-méthyl-2-oxo-5-phényl-1H-2,3-dihydro-1,4-benzodiazépine ou ses sels d'addition d'acide et ses isomères optiques.

**6.** Procédé selon la revendication 2, caractérisé en ce qu'on prépare des composés dans lesquels $R^2$ représente un atome d'hydrogène, un groupe alcoxy ayant 1 à 5 atomes de carbone, notamment méthoxy, un groupe alkyle ayant 1 à 5 atomes de carbone, notamment méthyle,ou un atome

d'halogène, en particulier le chlore, et $R^3$ représente un atome d'hydrogène; $R^4$ représente un groupe cycloalkyle ayant 5 ou 6 atomes de carbone ou un groupe thiényle , et $R^6$ représente un atome d'hydrogène ou d'halogène.

**7.** Procédé selon la revendication 6, caractérisé en ce qu'on prépare des composés dans lesquels $R^4$ représente un groupe cyclohexyle.